(19) Europäisches Patentamt
European Patent Office
Office européen des brevets

(11) **EP 2 910 623 B1**

(12) **EUROPÄISCHE PATENTSCHRIFT**

(45) Veröffentlichungstag und Bekanntmachung des Hinweises auf die Patenterteilung:
**17.01.2018 Patentblatt 2018/03**

(51) Int Cl.:
*C10G 9/00* *(2006.01)*        *G05B 13/04* *(2006.01)*
*G05B 23/02* *(2006.01)*       *G05B 17/02* *(2006.01)*

(21) Anmeldenummer: **15155787.3**

(22) Anmeldetag: **19.02.2015**

(54) **Verfahren und Vorrichtung zur Korrektur einer Messung**

Method and device for correcting a measurement

Procédé et dispositif destinés à la correction d'une mesure

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.02.2014 AT 501292014**

(43) Veröffentlichungstag der Anmeldung:
**26.08.2015 Patentblatt 2015/35**

(73) Patentinhaber: **OMV REFINING & MARKETING GMBH**
**1020 Wien (AT)**

(72) Erfinder: **Bacher, Wolfgang**
**2440 Gramatneusiedl (AT)**

(74) Vertreter: **Sonn & Partner Patentanwälte**
**Riemergasse 14**
**1010 Wien (AT)**

(56) Entgegenhaltungen:
**CN-A- 101 286 059      DE-A1- 4 127 536**

• DOCHAIN ET AL: "State and parameter estimation in chemical and biochemical processes: a tutorial", JOURNAL OF PROCESS CONTROL, OXFORD, GB, Bd. 13, Nr. 8, 1. Dezember 2003 (2003-12-01), Seiten 801-818, XP027107193, ISSN: 0959-1524 [gefunden am 2003-12-01]
• Kadlec, Gabrys: "Local Learning-Based Adaptive Soft Sensor for Catalyst Activation Prediction", Wiley Online Library, 17. August 2010 (2010-08-17), XP002740950, Gefunden im Internet: URL:https://s3.amazonaws.com/objects.readc ube.com/articles/downloaded/wiley/a6b78837 c78d14e8c41e75255b93d41527dc75c810ff0bec5 c d7a7192be27924.pdf?AWSAccessKeyId=AKIAIJ ZY FKH6APDFT3HA&Expires=1434585600&Signatu re= VFEgcwhp20eGlkW6tKLWJbPYiqA%3D&respon se-co ntent-type=application%2Fpdf [gefunden am 2015-06-18]
• Tatiraju, Soroush: "Nonlinear State Estimation in a Polymerization Reactor", American Chemical Society, 1. Januar 1997 (1997-01-01), XP002740951, Gefunden im Internet: URL:http://pubs.acs.org/doi/pdf/10.1021/ie 960905e [gefunden am 2015-06-18]

**Beschreibung**

Die Erfindung betrifft ein Verfahren und eine Vorrichtung zur Korrektur einer Messung einer Eingangsgröße eines chemischen Verarbeitungsprozesses zur Herstellung eines aus mehreren Produktkomponenten zusammengesetzten Produkts, wobei mit Messmitteln zur Messung der Eingangsgröße und zur Messung der Anteile mehrerer Produktkomponenten am Produkt während des Verarbeitungsprozesses die Eingangsgröße und die Anteile mehrerer Produktkomponenten am Produkt gemessen werden und wobei die Messmittel mit Rechenmitteln verbunden sind, mithilfe derer ein Korrekturwert der Eingangsgröße anhand der gemessenen Anteile ermittelt und für zukünftige Messungen gespeichert wird.

Insbesondere betrifft die Erfindung die Verbesserung der Vorhersagegüte von Vorhersagemodellen, welche im Zusammenhang mit petrochemischen Verarbeitungsprozessen eingesetzt werden, indem die Eingangsgröße(n) in das Modell bzw. deren Messwerte korrigiert werden. Die Korrekturen enthalten dabei im Idealfall sowohl reine Messfehler als auch Fehler, die sich aus den Umständen der Messung ergeben, z.B. wenn eine Eingangsgröße im realen Prozess an einer anderen Stelle gemessen wird als im Modell erwartet bzw. angenommen. Ein Beispiel für einen solchen chemischen Verarbeitungsprozess wäre ein Dampfspaltungsprozess, wobei das Produkt ein Spaltgas ist und die Produktkomponenten Spaltgaskomponenten sind. Als Eingangsgrößen kommen sämtliche Betriebsparameter des Verarbeitungsprozesses, welche eine Auswirkung auf die Produktzusammensetzung haben, in Frage; im Beispiel eines Dampfspaltungsprozesses wären derartige Eingangsgrößen eine Spaltgastemperatur an einem Spaltrohr-Austritt (Coil-Outlet-Temperature; COT), ein Spaltgasdruck am Spaltrohr-Austritt (COP), eine Einsatzmenge an zu spaltenden Kohlenwasserstoffen an einem Spaltrohr-Eintritt (FLOWR), eine Einsatzzusammensetzung, ein Verhältnis eines Prozessdampf-Volumens zur Einsatzmenge (D/KW), usw..

Häufig wird bei chemischen Verarbeitungsprozessen nur eine Eingangsgröße vollständig automatisch geregelt, so dass vor allem eine Korrektur der Messung dieser einen Eingangsgröße von Interesse ist. Bei Dampfspaltungsprozessen wird etwa in der Praxis oft nur die COT vollautomatisch geregelt, wobei die gemessene COT mit den gemessenen Anteilen von Propylen (P) und Ethylen (E) bzw. deren Verhältnis (P/E) in Beziehung gesetzt wird und von einer Abweichung des gemessenen P/E von dem aufgrund der gemessenen COT erwarteten P/E auf einen Fehler bei der Messung der COT geschlossen wird. Konkret wird dabei z.B. für einen Einsatz und einen Betriebspunkt eine "Kurve" P/E = f(COT) erstellt und aus dieser Kurve abgelesen, welcher COT dem gemessenen P/E entspricht. Die Differenz zwischen der so ermittelten COT und der gemessenen COT ergibt den Korrekturwert für die COT. Da der P/E Wert nicht nur von der COT abhängt, sondern auch von anderen - ebenfalls fehlerbehafteten - Betriebsparametern, vor allem auch von der Einsatzzusammensetzung, kann bei diesem Verfahren nur eine ungenaue Korrektur der Messung erreicht werden. Demzufolge ist die tatsächliche COT, d.h. in der Form, in der sie in das Vorhersagemodell eingeht, nur mit großer Ungenauigkeit bekannt. Insbesondere versagt diese Art der Korrekturbestimmung, wenn die tatsächliche Einsatzzusammensetzung von der der besagten "Kurve" (P/E = f(COT)) zugrunde liegenden Einsatzzusammensetzung abweicht.

Wenn die gemessene COT nun für Vorhersagen betreffend die Produktzusammensetzung herangezogen wird, pflanzt sich diese Ungenauigkeit naturgemäß auf sämtliche Vorhersagen fort, so dass beispielsweise eine Optimierung bereits durch die Größenordnung der resultierenden Vorhersagefehler begrenzt ist. Mit anderen Worten kann innerhalb des Fehlerbereichs der vorhergesagten Produktzusammensetzung, welcher aus dem Messfehler der Eingangsgröße resultiert, keine sinnvolle Optimierung vorgenommen werden.

Eine genaue Bestimmung des Messfehlers bzw. des Korrekturwerts wäre nur dann erzielbar, wenn alle anderen auf die Produktzusammensetzung einwirkenden Parameter exakt bekannt wären, so dass deren Einfluss bei der Ableitung des Korrekturwerts aus der Abweichung der Produktzusammensetzung explizit berücksichtigt werden könnte. Dies ist jedoch in der Praxis nicht möglich. Daher wird oft versucht, den Fehler durch Erhöhung der Anzahl der Messpunkte, z.B. durch oftmalige Messung der Anteile der Produktkomponenten, zu reduzieren. Dies kann jedoch das oben geschilderte grundsätzliche Problem nicht lösen sondern allenfalls stochastische (keinesfalls aber systematische) Fehler bei der Messung verringern.

Hinzu kommt, dass in der Praxis häufig nicht ein einzelner Verarbeitungsprozess, sondern mehrere parallel laufende Verarbeitungsprozesse in mehreren parallelen Kolonnen stattfinden. So werden bei einer Dampfspaltungsanlage in der Regel mehrere Spaltrohre parallel betrieben, wobei jedoch nur die Zusammensetzung des gesamten Produkts aller Spaltrohre gemessen wird. Die gemessenen Anteile sind somit bereits Mittelwerte über mehrere parallele Verarbeitungsprozesse bzw. die damit jeweils erzielten Anteile. Dementsprechend kann von diesem Mittelwert auch nur bestenfalls ein mittlerer Korrekturwert für die Messungen der Eingangsgröße abgeleitet werden. Da der Zusammenhang der zu korrigierenden Eingangsgröße mit den gemessenen Anteilen jedoch im Allgemeinen nicht-linear ist, wird durch die Mittelung ein zusätzlicher Fehler in den Korrekturwert eingeführt (dies folgt aus der Jensenschen Ungleichung).

Diese Probleme, d.h. die starke Abhängigkeit der Parameter der Regelstrecke (des Verarbeitungsprozesses) vom aktuellen Betriebspunkt sowie ihre Nichtlinearität und Komplexität, wurden bereits in DE 41 27 536 A1 erkannt. Bei dem darin gezeigten Verfahren zur Regelung einer Mehrkomponenten-Rektifikationskolonne werden die aktuell gemessenen Konzentrationen zweier Produktströme zur Feststellung etwaiger Regelabweichungen linear in Größen transformiert, die nach einem ebenfalls linearen Simulationsmodell jeweils nur von einer Eingangsgröße abhängen. Die Verwendung eines nichtlinearen Modells der Regelstrecke wird ausdrücklich verworfen und als für die quantitative Regelung ungeeignet bezeichnet. Eine Korrektur der Messung einer Eingangsgröße ist in DE 41 27 536 A1 ebenfalls nicht gezeigt, vermutlich weil eine solche Messung in der gezeigten Regelung praktisch überflüssig wird. Davon abgesehen kann dieses Verfahren selbstverständlich nur dann eingesetzt werden, wenn sowohl das Modell als auch der Arbeitsbereich der Anlage eine lineare Näherung zulässt. Dies ist im Allgemeinen nur dann der Fall, wenn die durch die Näherung eingeführten Fehler deutlich kleiner sind als das angestrebte Optimierungspotential, welches vorzugsweise Optimierungen im einstelligen Prozentbereich der Produktanteile umfasst. In allen anderen Fällen, d.h. bei grundlegend nichtlinearen Prozessen und entsprechenden nicht-linearen Modellen und bei Arbeitsbereichen (im Folgenden auch als Betriebsfenster oder "operating windows" bezeichnet), welche den Möglichkeiten der Anlage entsprechen, muss bei dem bekannten Verfahren der durch die Linearisierung gemachte Fehler über Feedback mit in Relation zur Prozessgeschwindigkeit ausreichender Frequenz und Genauigkeit ausgeglichen werden. Unter diesen Voraussetzungen ist der Nutzen aus der Transformation der gemessenen Komponenten jedoch gering, da aufgrund des raschen Feedbacks ohnehin jede Art von Fehlern zeitgerecht korrigiert werden kann. Eine Vorhersage über nicht realisierte Arbeitspunkte (bei denen naturgemäß kein Feedback zur Verfügung steht) ist bei diesem Verfahren nicht möglich bzw. nur mit inakzeptabler Genauigkeit. Dies liegt vor allem daran, dass hier nur eine linearisierte und nur lokal gültige Version des Modells in unabhängige Größen transformiert wird.

Die CN 101 286 059 A beschreibt allgemein ein Verfahren zur Messung und Korrektur von Daten betreffend Edukte und Produkte eines chemischen Verarbeitungsprozesses, wobei allerdings die Grundlage der Korrektur, d.h. auf Basis welcher Parameter die Korrektur durchgeführt wird, nicht genauer dargelegt ist.

Weiters befassen sich Dochain et al. in ihrem Artikel "State and parameter estimation in chemical and biochemical processes: a tutorial" (Journal of Process Control, Oxford, GB, Band 12, Nr. 8, 1. Dezember 2003, Seiten 801-818) mit den theoretischen Grundlagen der Schätzung von nicht direkt messbaren Prozessparametern bei chemischen und biochemischen Prozessen.Es ist Aufgabe der Erfindung, eine möglichst präzise Korrektur der Eingangsgröße auf Basis der realistisch verfügbaren Informationen, insbesondere der gemessenen Produktzusammensetzung, zu erzielen, welche möglichst unabhängig von weiteren Einflussfaktoren, welche die Produktzusammensetzung beeinflussen, ist und dementsprechend eine möglichst universale, d.h. von einem Arbeitspunkt des Verarbeitungsprozesses unabhängige, Gültigkeit besitzt. Dabei soll zugleich die Vorhersagequalität eines Vorhersagemodells auf Basis der Eingangsgröße verbessert werden und eine Verfälschung des auf Basis des Vorhersagemodells ermittelten optimalen Betriebspunkts minimiert werden.

Diese Aufgabe wird bei einem Verfahren bzw. einer Vorrichtung der eingangs angeführten Art erfindungsgemäß durch das Verfahren gemäß Anspruch 1 und die Vorrichtung gemäß Anspruch 14 gelöst.

Die Erfindung beruht dabei auf der allgemeinen Erkenntnis, dass die Genauigkeit der Korrektur einer Eingangsgröße eines Mehrgrößensystems, d.h. einer Regelstrecke mit mehreren Eingangs- und Ausgangsgrößen, dadurch verbessert werden kann, dass die Korrektur auf Basis einer virtuellen Ausgangsgröße ermittelt wird, welche einerseits eine Funktion der (realen und somit messbaren) Ausgangsgrößen ist und zugleich (beinahe) ausschließlich von einer einzelnen, nämlich der zugeordneten Eingangsgröße abhängt und somit unempfindlich gegenüber Änderungen der anderen - im Allgemeinen unbekannten - Eingangsgrößen ist. Dies gilt insbesondere für stark gekoppelte Systeme, bei denen jede Ausgangsgröße von mehreren Eingangsgrößen abhängt. Eine solche virtuelle Ausgangsgröße setzt sich demzufolge aus zumindest drei realen, d.h. gemessenen, Ausgangsgrößen zusammen und entspricht jener Abbildung dieser drei Ausgangsgrößen auf die zugeordnete Eingangsgröße, die den geringsten Einfluss anderer als der zugeordneten Eingangsgröße aufweist. Auch wenn prinzipiell mit nur zwei gemessenen Ausgangsgrößen eine Korrektur einer oder mehrerer Eingangsgrößen ermittelt werden könnte, sind die auf diese Weise ermittelten Korrekturwerte zwangsläufig korreliert, so dass eine unabhängige Zuordnung eines Fehlers praktisch nicht möglich ist. Auf der anderen Seite kann durch eine Abbildung von mehr als drei Ausgangsgrößen auf eine virtuelle Ausgangsgröße ein entsprechender Korrekturwert noch genauer und robuster bestimmt werden, so dass es im Extremfall auch sinnvoll ist, nur einen Korrekturwert und Verwendung aller verfügbaren gemessenen Ausgangsgrößen zu bestimmen. Der Übergang von realen, d.h. direkt gemessenen, und stark gekoppelten Ausgangsgrößen zu den virtuellen Ausgangsgrößen entspricht faktisch einer Entkopplung der Übertragungswege. Bei rein linearen Abhängigkeiten zwischen den Eingangsgrößen würde der Übergang bzw. - im mathematischen Sinne - einer (zumindest teilweisen) Diagonalisierung der Übertragungsmatrix des Mehrgrößensystems entsprechen. Die vorliegende Erfindung geht jedoch einen Schritt weiter, indem die virtuellen Ausgangsgrößen aufgrund der nicht-linearen Abbildung der Eingangsgrößen auch nicht-lineare Abhängigkeiten zwischen den Ausgangsgrößen auflösen bzw. entkoppeln können: wenn nur die virtuellen Ausgangsgrößen betrachtet werden, existiert im Idealfall im gesamten Arbeitsbereich des Verarbeitungsprozesses eine einfache Abhängigkeit der virtuellen Ausgangsgrößen von jeweils nur einer Eingangsgröße. Somit ist die Erfindung, auch wenn sie hier im Zusammenhang mit einem chemischen Verarbeitungsprozess, bei dem die Anteile der Produktkomponenten als Ausgangsgrößen angesehen werden, genauer beschrieben ist, prinzipiell auf beliebige Mehrgrößensysteme anwendbar.

Mit anderen Worten betrifft die Erfindung daher insbesondere ein Verfahren zur Messung und Korrektur einer Eingangsgröße eines chemischen Verarbeitungsprozesses zur Herstellung eines aus mehreren Produktkomponenten zusammengesetzten Produkts, wobei der Verarbeitungsprozess von zumindest zwei Eingangsgrößen abhängt, umfassend die Schritte, dass während des Verarbeitungsprozesses die Eingangsgröße gemessen wird, die Anteile mehrerer Produktkomponenten am Produkt gemessen werden, und ein Korrekturwert der Eingangsgröße anhand der gemessenen Anteile ermittelt und für zukünftige Messungen gespeichert wird. Erfindungsgemäß werden sodann insbesondere die Anteile zumindest dreier Produktkomponenten gemessen, und es wird der Korrekturwert aus der gemessenen Eingangsgröße und einer ihr zugeordneten nicht-linearen Abbildung der zumindest drei gemessenen Anteile ermittelt, wobei die Abbildung so gewählt ist, dass ihr Ergebnis mit der zu korrigierenden Eingangsgröße stärker korreliert als mit den übrigen Eingangsgrößen des Verarbeitungsprozesses.

Die Anwendung bei chemischen Verarbeitungsprozessen, d.h. bei der Umwandlung von Produkten bzw. Produktkomponenten, hat sich als besonders vorteilhaft herausgestellt, da hier in der Regel eine besonders starke Kopplung der Produktkomponenten vorliegt. Die zumindest drei Produktkomponenten, deren Anteile in die virtuelle Komponente eingehen bzw. die die Funktionsargumente der nicht-linearen Abbildung sind, sind vorzugsweise möglichst unabhängige, unterschiedliche Produktkomponenten und resultieren aus zumindest drei unabhängigen Messungen bzw. einer Messung mit zumindest drei Freiheitsgraden. Mit dem Anteil einer Produktkomponente ist in diesem Kontext eine absolute oder relative Menge der Produktkomponente, insbesondere ein Volumenanteil oder ein Massenanteil, gemeint. Auch wenn grundsätzlich bereits eine Verknüpfung der Anteile von zwei Produktkomponenten einer virtuellen Komponente entsprechen würde, ist es in der Praxis sinnvoll, die Anteile von zumindest drei Produktkomponenten zu berücksichtigen. Der durch die dritte Produktkomponente eingeführte Freiheitsgrad dient dabei der Normierung der Anteile, sodass die virtuelle Komponente unabhängig von einer absoluten Produktmenge ermittelt werden kann. Wenn man den für die Normierung verwendeten Freiheitsgrad abzieht, bleibt somit bei drei gemessenen Anteilen effektiv nur die Mindestzahl von zwei Freiheitsgraden für die virtuelle Komponente übrig.

Da der Zusammenhang zwischen Eingangsgrößen und Produktkomponenten im Allgemeinen nicht ohne Weiters invertierbar ist, kann zur Ermittlung des Korrekturwerts vorteilhafter Weise ein erwarteter Anteil der virtuellen Komponente ausgehend von der gemessenen Eingangsgröße und einem Vorhersagemodell des Verarbeitungsprozesses ermittelt werden und der Korrekturwert aus der Abweichung des aktuellen Anteils der virtuellen Komponente von dem erwarteten Anteil der virtuellen Komponente ermittelt werden. Insbesondere kann zumindest lokal ein linearer Zusammenhang zwischen der Eingangsgröße und der virtuellen Komponente abgeleitet und damit aus der Abweichung der entsprechende Korrekturwert berechnet werden.

Wenn zumindest zwei Eingangsgrößen gemessen werden und jeweils ein Korrekturwert ermittelt wird, wobei jeder Eingangsgröße eine eigene virtuelle Komponente entsprechend einer individuellen nicht-linearen Abbildung zugeordnet ist, kann vorteilhafterweise eine unabhängige und somit rasche Korrektur der Messungen beider Eingangsgrößen realisiert werden. Vorzugsweise wird die Anzahl der gemessenen Anteile die Anzahl der daraus insgesamt ermittelten, unabhängigen Korrekturwerte immer um eins übersteigen. Dies bedeutet jedoch nicht, dass die Anzahl der in der Praxis ermittelten Korrekturwerte nicht die Anzahl der gemessenen Eingangsgrößen übersteigen kann, da die Korrekturwerte untereinander im Allgemeinen nicht unabhängig sind. Deren Abhängigkeiten sind naturgemäß implizit in den virtuellen Komponenten abgebildet und werden daher bei der Korrektur abhängiger Eingangsgrößen berücksichtigt. Es kommt lediglich darauf an, dass die durch die virtuellen Komponenten zwischen Eingangsgrößen und virtuellen Ausgangsgrößen definierte nicht-lineare Abbildung bestimmt oder vorzugsweise überbestimmt ist.

Neben der Korrektur der Messungen und der dadurch erzielten Verbesserung der Qualität der auf Basis dieser Messungen durchgeführten Vorhersagen, ist es vorteilhaft, wenn eine der Eingangsgröße entsprechende Stellgröße des Verarbeitungsprozesses geregelt wird, wobei eine Regelabweichung der Stellgröße in Abhängigkeit von dem ermittelten und gespeicherten Korrekturwert der Eingangsgröße ermittelt wird. Dies ist selbstverständlich auf mehrere Eingangsgrößen und mehrere Stellgrößen analog anwendbar. Auf diese Weise kann eine Übereinstimmung zwischen den Eingangsgrößen des Vorhersagemodells und den Führungsgrößen der Prozessregelung erzielt und die Regelung somit vereinfacht werden.

Um eine vom Arbeitspunkt des Verarbeitungsprozesses (als Arbeitspunkt bzw. Betriebspunkt sei hier die Gesamtheit der Werte der Betriebsparameter, einschließlich sämtlicher Eingangsgrößen definiert) möglichst unabhängige Ermittlung des Korrekturwerts zu erzielen, ist es vorteilhaft, wenn die virtuelle Komponente, zumindest innerhalb eines Betriebsfensters mehrerer Eingangsgrößen (welches dem Arbeitsbereich des Verarbeitungsprozesses entspricht), im Wesentlichen nur von einer einzigen Eingangsgröße abhängig und dieser zugeordnet ist bzw. der Einfluss der übrigen Eingangsgrößen auf die virtuelle Komponente kleiner ist als jener der jeweils zugeordneten Eingangsgröße. Das Betriebsfenster bzw. der Arbeitsbereich ist hierbei durch die im Betrieb erwarteten oder vorhergesehenen Parameterbereiche der Betriebsparameter bzw. der Eingangsgrößen definiert.

Weiters ist es günstig (aber nicht notwendig), wenn zwei der Anteile auf den ersten Anteil bzw. mithilfe des ersten Anteils normiert werden. Im einfachsten Fall gehen der zweite und jeder weitere Anteil nur als Verhältnisse zum ersten Anteil in die virtuelle(n) Komponente(n) ein. Diese Vorgehensweise hilft dabei, Probleme bei der Probennahme und Spaltgasanalyse zu vermindern. Außerdem eignet sich die Verwendung von, insbesondere ganzzahligen, Verhältnissen zur Abbildung der stöchiometrischen Verhältnisse bei den Gleichgewichtsreaktionen, welche ein natürlicher Teil des Verarbeitungsprozesses sind.

Weiters ist es günstig, wenn die gemessenen Anteile mit einem jeweils zugeordneten reellen Exponenten in die virtuelle(n) Komponente(n) eingehen. Der durch die im Allgemeinen von eins abweichenden Exponenten festgelegte nicht-lineare Einfluss der einzelnen Anteile auf die virtuelle Komponente eignet sich erfahrungsgemäß besonders gut zur Modellierung der beobachteten Verarbeitungsprozesse. Die potenzierten Anteile bilden dabei eine Basis von nicht-linearen Grundfunktionen. Diese Grundfunktion sind bevorzugt Multiplikationen von mehreren potenzierten Anteilen, wobei sowohl positive als auch negative Exponenten verwendet werden, welche Exponenten dem Betrag nach nicht identisch sein müssen. Prinzipiell können auch andere nicht-lineare Grundfunktionen, wie Hyperbelfunktionen, die Exponentialfunktion oder Logarithmen eingesetzt werden. Eine möglichst breite Palette von Grundfunktionen ermöglicht einer nachfolgenden Anpassung (z.B. durch lineare Regression, s.u.) die Auswahl der geeigneten Funktionen automatisch zu treffen.

In diesem Zusammenhang kann vorteilhafter Weise die nicht-lineare Abbildung einer Linearkombination von nicht-linearen Grundfunktionen entsprechen, wobei jeder Grundfunktion durch lineare Regression ein geeigneter Koeffizient zur Gewichtung der Grundfunktion zugewiesen wird; insbesondere werden die Koeffizienten der nicht-linearen Abbildung(en) vorzugsweise durch multivariable lineare Regression, ausgehend von einem Vorhersagemodell des chemischen Verarbeitungsprozesses, insbesondere zur Approximation des Vorhersagemodells innerhalb eines vorgesehenen Betriebsfensters des Verarbeitungsprozesses, bestimmt. Die lineare Regression wählt dabei automatisch jene Linearkombination der nicht-linearen Grundfunktionen aus, die in Summe ein möglichst lineares Verhalten, d.h. einen möglichst linearen Zusammenhang zwischen der resultierenden virtuellen Komponente und der jeweils zugeordneten Eingangsgröße, ergeben. Falls diese Möglichkeit besteht, d.h. wenn ein entsprechend präzises Modell des Verarbeitungsprozess zur Verfügung steht, kann auf diesem Weg eine rigorose und nachvollziehbare Ableitung der nicht-linearen Abbildung, im Idealfall sogar einer generischen Formulierung der nicht-linearen Abbildung, erfolgen. Als Regression wird in diesem Zusammenhang jedes Verfahren bezeichnet, bei dem eine Funktion y mittels Minimierung der Fehlerquadrate angenähert wird. Wie die Funktion aussieht, ist nicht beschränkt. Im Fall einer linearen Regression, d.h. $y = X \cdot b$, ist X ist eine Matrix und b ein Spaltenvektor. Wie die Spalten in X wiederum zustande kommen spielt dabei für die Regression keine Rolle. In diesem Sinne wird eine lineare Regression daher auch immer einen linearen Zusammenhang bieten. Die Nicht-Linearität der die virtuelle Komponente definierenden Abbildung ergibt sich aus der Nicht-Linearität der Grundfunktionen. Für eine Approximation des Vorhersagemodells innerhalb eines vorgesehenen Betriebsfensters des Verarbeitungsprozesses wird die Regression im gesamten Betriebsfenster durchgeführt; die resultierenden nicht-linearen Abbildungen approximieren die Zusammenhänge des Vorhersagemodells im gesamten Betriebsfenster daher im Durchschnitt gleich gut, d.h. es wird in der Regression kein Betriebspunkt bevorzugt oder benachteiligt (es werden alle Betriebspunkte gleich gewichtet). Alternativ oder zusätzlich kann es Bereiche innerhalb des Betriebsfensters geben, in denen mittels lokaler Bestimmung der virtuellen Komponente die Approximation des Vorhersagemodells lokal verbessert werden kann. Als Betriebsfenster wird hierbei ein begrenzter, n dimensionaler Parameterraum bezeichnet, wobei n der Anzahl jener Eingangsgrößen entspricht, welche als Betriebsparameter zusammen eine Konfiguration bzw. einen Arbeitspunkt des Verarbeitungsprozesses definieren. Die Grenze (d.h. die n-1 dimensionale Grenzfläche) des Betriebsfensters ist dabei durch physikalische Limitierungen und die Möglichkeiten und Grenzen einer den Verarbeitungsprozess realisierenden Anlage definiert. Vereinfacht entspricht das Betriebsfenster der Kombination der im Betrieb erwarteten oder vorhergesehenen Parameterbereiche der Betriebsparameter.

Der Einfachheit halber bzw. wenn das verfügbare Modell nur numerisch ausgewertet werden kann, können die Koeffizienten der Abbildung alternativ aus einem Kennfeld der erwarteten Produktzusammensetzungen aller möglichen bzw. zumindest aller vorausgesehenen Eingangsgrößenkonfigurationen innerhalb eines Betriebsfensters des Verarbeitungsprozesses abgeleitet werden, wobei das Kennfeld aus dem Vorhersagemodell des Verarbeitungsprozesses generiert wird.

Es ist außerdem besonders günstig, wenn bei der Ableitung der Koeffizienten alle im Rahmen des Verarbeitungsprozesses gemessenen bzw. messbaren Anteile von Produktkomponenten berücksichtigt werden. Dadurch kann eine optimale Basis für die Auswahl der berücksichtigten Produktkomponenten getroffen werden.

Weiters ist es vorteilhaft, wenn bei der Ermittlung der Koeffizienten mit den gemessenen Anteilen der Produktkomponenten assoziierte Messfehler berücksichtigt werden, indem vorzugsweise genauer messbaren Anteilen ein höheres Gewicht, d.h. ein größerer Einfluss auf die virtuelle Komponente, gegeben wird als weniger genau messbaren. Auf diese Weise kann der Einfluss von nur ungenau quantifizierbaren Anteilen entsprechend ihrer Genauigkeit reduziert werden, so dass die mit der virtuellen Komponente insgesamt assoziierte Genauigkeit möglichst hoch ist bzw. die assoziierte Unsicherheit möglichst gering.

Wenn der chemische Verarbeitungsprozess ein Dampfspaltungsprozess ist, wobei das Produkt ein Spaltgas ist und die Produktkomponenten Spaltgaskomponenten sind, insbesondere im Spaltgas enthaltene Kohlenwasserstoffe, kann mit dem erfindungsgemäßen Verfahren eine genaue Korrektur der Messungen der Eingangsgrößen und somit eine bessere Vorhersagequalität des Prozessmodells und letztlich eine bessere Ausnutzung der eingesetzten Ressourcen erzielt werden. Bei derartigen Prozessen bzw. den entsprechenden Anlagen (z.B. "Steamcrackern") wird häufig auf eine detaillierte Korrektur der Vielzahl von zugänglichen Eingangsgrößen verzichtet, weil es schwierig ist, aus den direkt gemessenen Eingangsgrößen ohne Weiteres auf die jeweiligen Korrekturwerte zu schließen. Mit dem hier vorgeschlagenen Verfahren können trotz der komplizierten Zusammenhänge innerhalb der Regelstrecke und der starken Kopplung der Produktkomponenten zuverlässige und präzise Korrekturwerte, insbesondere auch für eine Vielzahl von Eingangsgrößen, ermittelt werden.

Konkret hat es sich in diesem Zusammenhang als vorteilhaft herausgestellt, wenn die mithilfe des erfindungsgemäßen Verfahrens korrigierte(n) Eingangsgröße(n) eine Spaltgastemperatur an einem Spaltrohr-Austritt bzw. an einem Ofenaustritt, ein Spaltgasdruck am Spaltrohr-Austritt bzw. am Ofenaustritt, eine Einsatzmenge an zu spaltenden Kohlenwasserstoffen an einem Spaltrohr-Eintritt, eine Einsatzzusammensetzung und/oder ein Verhältnis eines Prozessdampf-Volumens zur Einsatzmenge (D/KW oder auch "Dilution Steam", DS) ist bzw. umfassen. Insbesondere bei der Spaltgastemperatur, dem Spaltgasdruck und dem D/KW-Wert können mithilfe des vorliegenden Verfahrens gezielte Änderungen auch mehrerer Parameter gleichzeitig auf einfache Weise ermittelt werden bzw. können Abweichungen der tatsächlich wirkenden Eingangsgrößen von den gemessenen Eingangsgrößen rasch erkannt und korrigiert werden.

Die Erfindung wird nachfolgend anhand von besonders bevorzugten Ausführungsbeispielen, auf die sie jedoch nicht beschränkt sein soll, und unter Bezugnahme auf die Zeichnung noch weiter erläutert. In den Zeichnungen zeigen dabei im Einzelnen:

Fig. 1 schematisch und stark vereinfacht einen heißen Teil eines Steamcrackers mit einer erfindungsgemäßen Vorrichtung;
Fig. 2 vier Diagramme untereinander zur Darstellung der Betriebspunkte eines Ausgangszustands anhand von vier Eingangsgrößen eines Ofens gemäß Fig. 1 mit zwei Spaltrohren;
Fig. 3 fünf Diagramme der Massenanteile von fünf Spaltgaskomponenten bei einem Betrieb entsprechend den in Fig. 2 dargestellten Betriebspunkten;
Fig. 4 vier Diagramme wie in Fig. 2, wobei die Betriebspunkte mit Fehlern entsprechend sechs verschiedenen Fehlersituationen versehen sind, wobei die Fehler in den beiden Spaltrohren identisch sind;
Fig. 5 vier Diagramme gemäß Fig. 4, wobei die Fehler in den beiden Spaltrohren unabhängig sind;
Fig. 6 sechs Diagramme für sechs verschiedene Spaltgaskomponenten, wobei jedes Diagramm zum Vergleich die Vorhersagefehler dreier Methoden zur Ermittlung von Korrekturwerten für die in Fig. 4 dargestellten Betriebspunkte angibt; und
Fig. 7 sechs Diagramme der Vorhersagefehler gemäß Fig. 6 für die in Fig. 5 dargestellten Betriebspunkte.

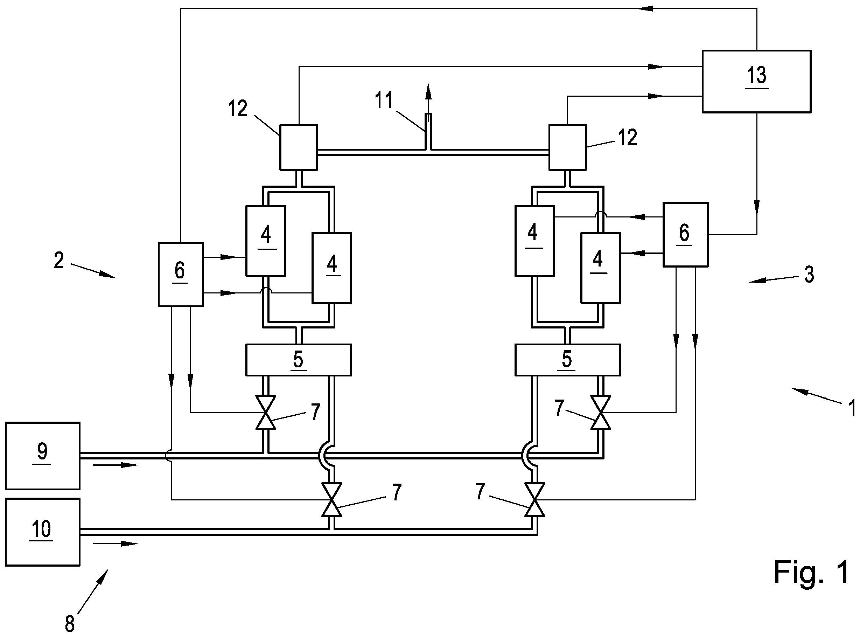

In Fig. 1 ist ein Steamcracker 1 mit zwei Öfen 2, 3 dargestellt. Die beiden Öfen 2, 3 weisen jeweils zwei Spaltrohre 4 auf, welche mit einem Ofeneinsatz 5 verbunden sind. Die Stellgrößen der Spaltrohre 4 werden von einer Steuerungseinheit 6 des jeweiligen Ofens 2, 3 vorgegeben. Außerdem sind die Steuerungseinheiten 6 mit Ventilen 7 eines Einsatzsystems 8 des Steamcrackers 1 verbunden, so dass die Ofeneinsätze 5 aus den Einsätzen 9, 10 des Einsatzsystems 8 entsprechend den Ventileinstellungen gespeist werden und so die Einsatzzusammensetzung gesteuert werden kann. Zur besseren Übersichtlichkeit wurde von einer Darstellung des Prozessdampfsystems des Steamcrackers 1, mit welchem die Öfen 2, 3 ebenfalls verbunden sind, abgesehen. Die Ausgänge der Spaltrohre 4 sind verbunden und münden über einen Ofenaustritt des jeweiligen Ofens 2, 3 in eine gemeinsame Verbindungsleitung 11, welche das in den Spaltrohren 4 produzierte Spaltgas in einen warmen Teil (nicht dargestellt) des Steamcrackers 1 überführt. Mit der Verbindungsleitung 11 sind Messmittel 12 verbunden, welche die Anteile der Produktkomponenten im Spaltgas ermitteln, wobei jedem Ofen 2, 3 ein Messmittel 12 zugeordnet ist. Die Messmittel 12 sind eingerichtet, eine Spaltgaszusammensetzung jeweils am Ofenaustritt der einzelnen Öfen 2, 3 zu analysieren. Darüber hinaus können zwischen der Vereinigung der Ausgänge der Spaltrohre 4 und dem jeweiligen Messmittel 12, d.h. in oder entlang einer Verbindungsleitung zwischen den Spaltrohren 4 und den Messmitteln 12, weitere Apparate (nicht gezeigt), z.B. Wärmetauscher, angeordnet sein. In der Praxis kann somit die Spaltgaszusammensetzung und/oder der Spaltgasdruck entweder direkt am Ausgang des Spaltrohrs 4 (auch als Spaltrohr-Austritt bezeichnet), z.B. bei 850°C, oder auch erst nachdem das Spaltgas in einem Wärmetauscher (nicht gezeigt) auf 150-500°C, insbesondere 200-450°C, abgekühlt wurde, gemessen werden. Mit den Messmitteln 12 sind außerdem Rechenmittel 13 verbunden, welche die von den Messmitteln 12 ermittelten Anteile der Produktkomponenten empfangen und daraus Korrekturwerte für an den Öfen 2, 3 gemessene Eingangsgrößen der Öfen 2, 3 ermitteln. Die Rechenmittel 13 sind vorzugsweise mit den Steuerungseinheiten 6 der Öfen 2, 3 verbunden, um - ausgehend von den ermittelten Korrekturwerten - auf deren Stellgrößen, d.h. auf die Stellgrößen der Spaltrohre 4 sowie vorzugsweise auch die Ventileinstellungen, Einfluss zu nehmen.

Angenommen, es werden in dem Steamcracker 1 gemäß Fig. 1 z.B. die Spaltgaskomponenten $CH_4$, $C_2H_4$, $C_3H_6$ und $C_3H_8$ mittels Messung bestimmt, wobei die jeweilige Menge durch unterschiedliche Eingangsgrößen der Spaltrohre 4, wie Spaltgastemperatur an einem Spaltrohr-Austritt ("Coil Outlet Temperature", COT), ein Spaltgasdruck am Spaltrohr-Austritt ("Coild Outlet Pressure", COP), eine Einsatzmenge an zu spaltenden Kohlenwasserstoffen an einem Spaltrohr-Eintritt ("Flowrate", FLOWR), eine Einsatzzusammensetzung und/oder ein Verhältnis eines Prozessdampf-Volumens zur Einsatzmenge (D/KW bzw. DS) zustande kommt. Wenn beispielsweise 100 Messpunkte bekannt sind, können mit Hilfe des Modells SPYRO der Fa. Technip Benelux B.V. die Abhängigkeiten (Sensitivitäten) von den Eingangsgrößen auf die Spaltgaskomponenten berechnet werden.

Soll beispielsweise ein Korrekturwert für die Messung der COT (bzw. einer beliebigen anderen Eingangsgröße) bestimmt werden, müsste man alle auf die Spaltgaskomponenten einwirkenden Größen und Parameter exakt kennen - was realistisch nicht möglich ist - oder aber man muss eine Ausgangsgröße finden, die (nahezu) ausschließlich von der COT abhängt.

Die vorliegende Erfindung umfasst ein Verfahren, welches sich die zweite Möglichkeit zunutze macht. Ziel ist es, eine virtuelle Ausgangsgröße als Funktion der realen (messbaren) Ausgangsgrößen, d.h. der gemessenen Spaltgaskomponenten, zu finden, die beinahe ausschließlich von der zu bestimmenden Eingangsgröße (z.B. COT, FLOWR, D/KW, usw.) abhängt. Diese Größe wird in diesem Zusammenhang als virtuelle Komponente bezeichnet.

Es gibt naturgemäß beliebig viele Möglichkeiten, die virtuelle Komponente zu definieren. Die Erfahrung zeigt, dass es zumindest in diesem Anwendungsfall (d.h. bei der Bestimmung eines Korrekturwerts für die gemessene COT) problemlos möglich ist, den Anteil der Information z.B. der COT an der virtuellen Komponente auf beinahe 100 % zu erhöhen. D.h. eine solche virtuelle Komponente hängt im Wesentlichen ausschließlich von einer einzigen zu korrigierenden Eingangsgröße, d.h. in diesem Fall der COT, ab.

Einen sehr eleganten und schnellen Weg, um eine entsprechende virtuelle Komponente aufzufinden bzw. zu definieren, stellt die Verwendung einer Partial Least Squares (PLS) oder Lasso Regression dar. Diese Methoden liefern - vereinfacht gesagt - Informationen darüber, welche Größen besser als andere für die Verwendung in einer bzw. als Teil einer virtuellen Komponente geeignet sind. Mathematisch betrachtet ist das Ziel, eine Größe (die virtuelle Komponente) zu finden, die möglichst gut mittels Regression durch die gewünschte, d.h. die zu korrigierende, Eingangsgröße ausgedrückt werden kann.

Zur Ermittlung einer entsprechenden virtuellen Komponente definiert man zunächst eine Anzahl an Grundfunktionen, die jeweils Funktionen der Ausgangsgrößen, d.h. der gemessenen Spaltgaskomponenten bzw. genauer gesagt Funktionen der jeweils gemessenen Menge mehrerer Spaltgaskomponenten, sind. Diese Grundfunktionen sind zumindest teilweise nicht-lineare Funktionen der gemessenen Anteile von einer oder mehreren Produktkomponenten. Beispielsweise könnten die Grundfunktionen folgendermaßen aussehen:

$$f_1 = \frac{C2H4}{C3H6}, \quad f_2 = \frac{CH4}{C3H6}, \quad f_3 = \frac{CH4}{C3H8}, \quad f_4 = \frac{C2H4}{C3H8}, \quad \text{usw.} \qquad (1)$$

Die hier verwendete, vereinfachte Notation verwendet die chemischen Summenformeln als Platzhalter für den Anteil (d.h. die Menge) der jeweils durch die Summenformel beschriebene Spaltgaskomponente. In Gleichung (1) gibt der Wert der Grundfunktion $f_1$ beispielsweise das Verhältnis der Mengen von Ethen und Propen im Spaltgas an. Eine andere Möglichkeit für die Wahl der Grundfunktionen wäre z.B.

$$f_1 = C2H4, \quad f_2 = \left(C2H4\right)^2, \quad f_3 = C2H4 \cdot C3H6, \quad f_4 = \frac{\left(C3H6\right)^{\frac{1}{2}}}{C3H8}, \quad \text{usw.} \qquad (2)$$

Ausgehend von dem auf diese Art festgelegten Set an Grundfunktionen berechnet man nun mittels SPYRO ausgehend von den bekannten, aber möglicherweise fehlerbehafteten, Eingangsgrößen für ausreichend viele Spaltgaskomponenten, d.h. zumindest für die in den Grundfunktionen verwendeten Spaltgaskomponenten, obige Funktionen und führt diese Spaltenvektoren zu einer Prädiktor-Matrix zusammen. Der erste Vektor wird dabei auf eine beliebige Konstante festgelegt, um bei der Regression den "0-Punkt" zu definieren. Es ergibt sich somit folgende Matrix, wobei die Zeilen der Matrix unterschiedlichen Werten der Eingangsgrößen und somit im Allgemeinen unterschiedlichen Spaltgaszusammensetzungen entsprechen:

$$X = \left[ 1, f_1, f_2, f_3, f_4, \ldots \right] \qquad (3)$$

Die zugehörige COT entspricht in diesem Fall einem Response-Spaltenvektor:

$$y = \begin{bmatrix} \mathrm{COT} \end{bmatrix} \qquad\qquad (4)$$

Mittels Regression wird nun bestimmt, wie die Gewichtungen (lineare Superposition) der einzelnen Grundfunktionen aussehen müssen, um eine optimale Näherung der COT in Abhängigkeit von den vorgegebenen Grundfunktionen zu ermöglichen.

Es wird hierzu der Spaltenvektor b mittels linearer Regression so bestimmt, dass die Summe der quadrierten Fehler $y_{res}$ ("res" als Abkürzung für "residual") minimiert wird:

$$y = X \cdot b + y_{res} \, , \qquad\qquad (5)$$

wobei der Spaltenvektor b angibt, wie die einzelnen Grundfunktionen gewichtet aufsummiert werden müssen, um die gesuchte virtuelle Komponente zu erhalten. Zur Bestimmung der COT-Korrektur muss dann im Betrieb nur noch folgende Differenz berechnet werden:

$$\mathrm{COT}_{Korrektur} = X_{gemessen} \cdot b - X_{erwartet} \cdot b \, , \qquad\qquad (6)$$

wobei $X_{gemessen}$ genauso berechnet wird wie $X_{erwartet}$, aber anstelle der aufgrund der angenommenen Eingangsgrößen erwarteten Spaltgaskomponenten werden die gemessenen Spaltgaskomponenten eingesetzt. Die Korrekturwerte sind dabei nicht konstant, sondern ändern sich im Laufe der Zeit und zum Teil auch in Abhängigkeit des Einsatzes, d.h. der Einsatzzusammensetzung. Daher ist eine ständige Kontrolle und Anpassung nötig.

Bei der Wahl der Grundfunktionen gibt es theoretisch unendlich viele Möglichkeiten. Es ist zielführend, die Grundfunktionen derart zu wählen, dass $y_{res}$ minimal wird. Als Auswahlhilfe kann bei der Verwendung einer PLS Regression die Transformation des Spaltgaskomponentenraums auf die Scores verwendet werden, wie im Folgenden erklärt wird. Vereinfacht gesagt wird bei der PLS Regression sowohl der Eingangsraum als auch der Ausgangsraum mittels Principal Component Analysis (PCA) transformiert. Die Regression findet nun nicht direkt von dem Ein- zum Ausgangsraum statt, sondern über deren Transformationen. Die lineare PCA sorgt dafür, dass die im linearen Sinn wichtigen Informationsträger hervorgehoben werden, d.h. man erkennt, in welchen Grundfunktionen im Vergleich zu anderen überhaupt nennenswerte Information steckt. Dies kann im ersten Schritt zur Auswahl geeigneter Grundfunktionen dienen. Die Scores stellen die Transformation des Ein- bzw. Ausgangsraum dar und den kann man grafisch in z.B. 2D darstellen. Wenn man mit möglichst wenigen Grundfunktionen auskommen will, kann man nach transformierten Grundfunktionen suchen, die z.B. möglichst linear, bzw. die in Kombination möglichst linear sind (d.h. die Form einer Nichtlinearität kompensiert gut die Form einer anderen). Diese Auslese verbessert aber kaum das Endergebnis. Das Ziel der Auslese wäre es nur, die Anzahl an Grundfunktionen und somit den Rechenaufwand zu reduzieren, wobei das in der Praxis meist den Aufwand nicht wert ist. Für die vorliegende Erfindung ist die konkrete Auswahl der optimalen Grundfunktionen nicht entscheidend; das macht die Regression ganz von selbst.

Werden z.B. ausschließlich Verhältnisse der Anteile der Spaltgaskomponenten als Grundfunktionen bzw. in den Grundfunktionen verwendet, hat dies den Vorteil, dass eine durch die Messung bedingte fehlende 100%-Referenz nicht mehr ins Gewicht fällt, d.h. die absolute Menge an Spaltgas hat in diesem Fall keinen Einfluss auf die Korrektur der Eingangsgröße(n).

Die virtuelle Größe bzw. Komponente löst gleichzeitig teilweise das Problem, dass man bei Mittelung der Eingangsgrößen über mehrere Spaltrohre bedingt durch Nichtlinearitäten einen zusätzlichen Fehler begeht. Durch den hohen Informationsgehalt und bedingt durch die Regression ist die virtuelle Komponente in Bezug auf die COT (und die anderen Eingangsgrößen) so gut wie linear. Die Nichtlinearität verschwindet daher auf der Seite der Eingangsgrößen (COT, D/KW bzw. DS, COP, FLOWR usw.) und somit auch teilweise das Problem des zusätzlichen Fehlers bei der Mittelwertbildung aufgrund eines nichtlinearen Anteils.

Sollte bekannt sein, dass manche Spaltgaskomponenten genauer gemessen oder berechnet werden können als andere, kann dies durch Gewichtungen in der Regression berücksichtigt werden.

Obige Methodik lässt sich für jede die Spaltgaskomponenten beeinflussende Größe (d.h. jeden Modell-Parameter) anwenden. Die Sensitivität wird hierbei immer erhöht, d.h. die Bestimmung der Parameter-Anpassung deutlich verbessert.

Um die Vorteile des erfindungsgemäßen Verfahrens zu illustrieren, wurde ein Vergleich mit den im Stand der Technik bekannten und in der Praxis bisher eingesetzten Verfahren durchgeführt, dessen Ergebnisse im Folgenden erläutert werden.

Ausgangspunkt ist ein typischer Betriebspunkt eines Ofens vom Typ B9601 mit zwei Spaltrohren. Es wird mittels des Modells des geregelten Prozesses (SPYRO) für einen bestimmten Betriebspunkt der Eingangsgrößen (COT, FLOWR, DS, COP und Einsatzzusammensetzung) die Spaltgaszusammensetzung pro Ofen ermittelt. Der Betriebspunkt in beiden Spaltrohren wurde folgendermaßen definiert:

$$COT \quad = 850 \ °C,$$

$$DS \quad = 0,4,$$

$$FLOWR = 2700 \ kg/h/coil$$

und

$$COP \quad = 1,9 \ bar_{abs}.$$

Die Zusammensetzung des zu spaltenden Einsatzes ist ebenso wichtig, wird jedoch hier der Einfachheit halber als bekannt und konstant angenommen. Ausgehend von dem Betriebspunkt wird die Zusammensetzung pro Spaltrohr berechnet und anschließend pro Ofen gemittelt. Diese gemittelte Spaltgaszusammensetzung entspricht dem, was die Messmittel, d.h. die Spaltgasanalysatoren (die in der Praxis als Referenz dienen) messen.

In der Praxis liegt immer Prozessrauschen vor, weshalb den Eingangsgrößen ein normal verteiltes Rauschen hinzugefügt wurde. Die Rauschanteile (Gauß-verteilt, 1 Sigma) der jeweiligen Eingangsgrößen wurden folgendermaßen festgelegt:

$$COT \quad : 0,06 \ \%,$$

$$DS \quad : 0,3 \ \%,$$

$$FLOWR : 0,3 \ \%$$

und

$$COP \quad : 0,3 \ \%.$$

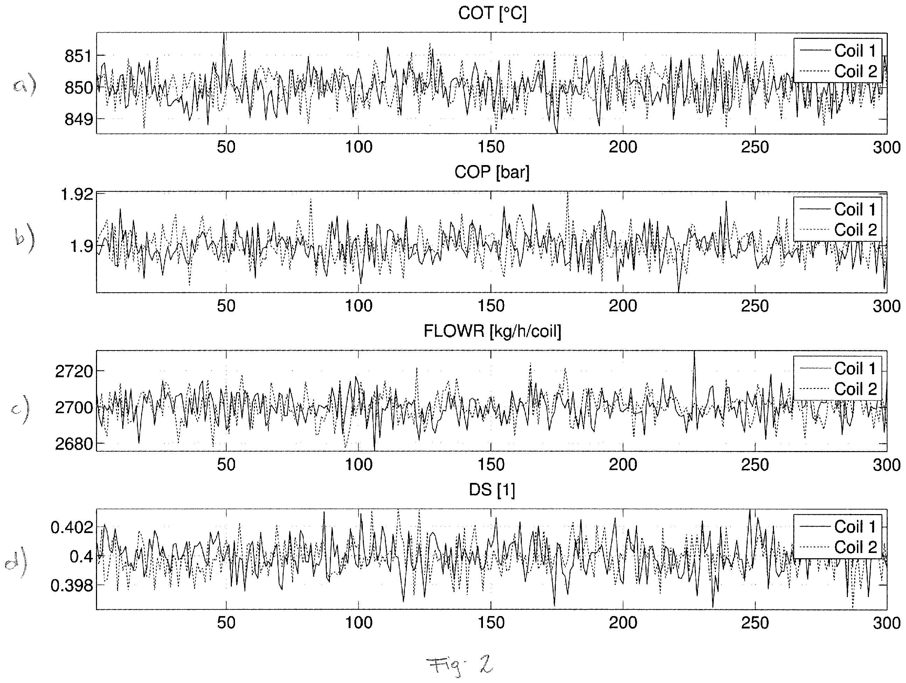

Ausgehend von diesen Verteilungen wurden 300 Parameter-Kombinationen, welche jeweils einem Betriebspunkt entsprechen, generiert, die den Ausgangszustand, d.h. abgesehen vom Prozessrauschen ohne Fehler oder Abweichungen, repräsentieren. Fig. 2 a)-d) zeigen die resultierenden Verteilungen der vier Eingangsgrößen in jeweils einem Liniendiagramm, wobei jeder Eingangsgröße für jedes der beiden Spaltrohre ein Parameter-Wert zugeordnet ist, was in den Diagrammen durch zwei separate Linien für "Coil 1" und "Coil 2" angedeutet ist.

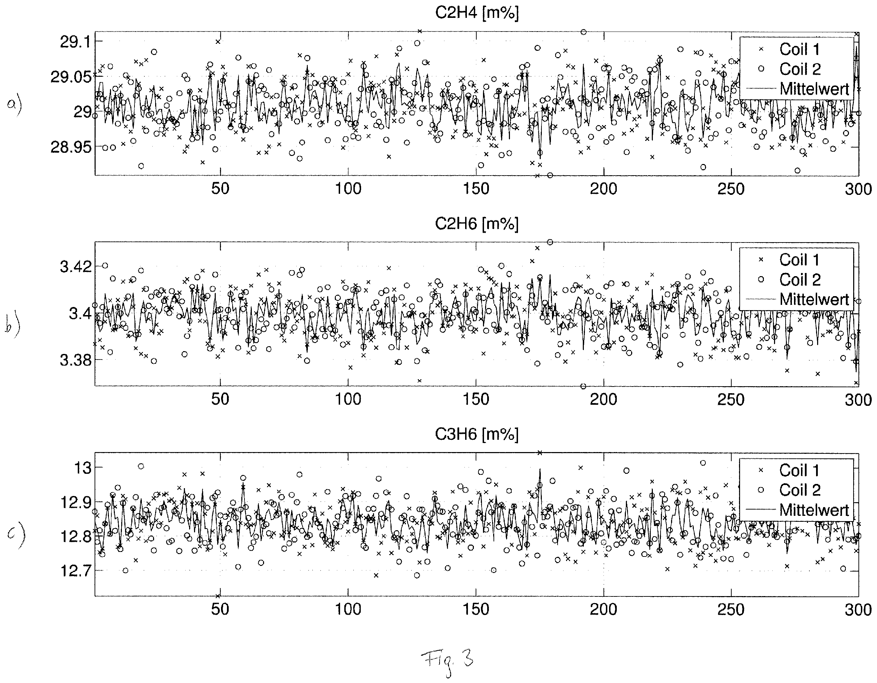

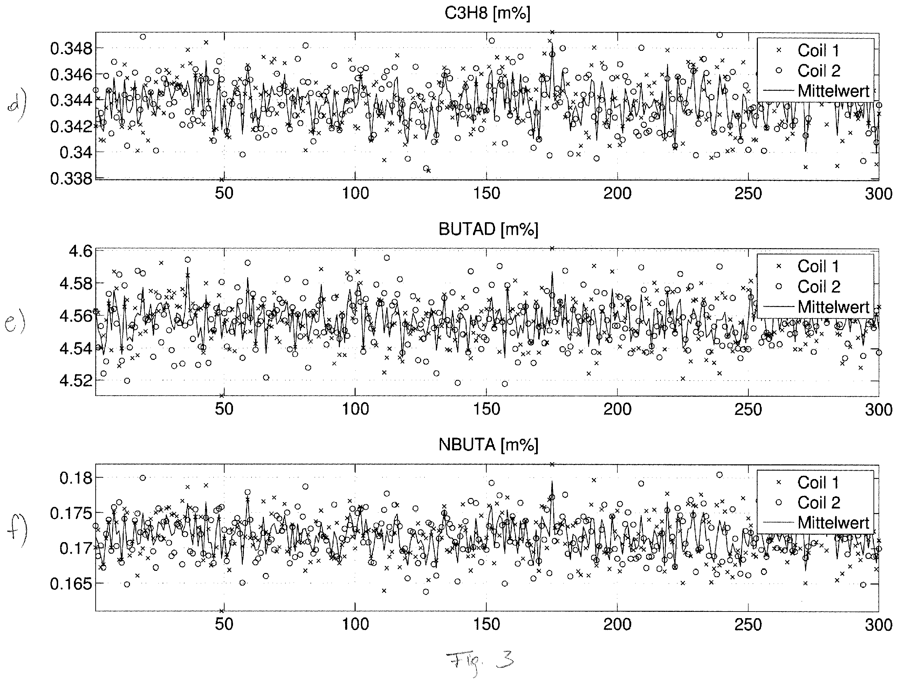

Für jeden der 300 Betriebspunkte wurden anschließend die Anteile von sechs für den optimalen Betrieb sehr wichtigen Spaltgaskomponenten ermittelt und in Fig. 3 a)-f) dargestellt. Die Anteile (an der Gesamtmasse, d.h. Massen-% bzw. "m%") nach den einzelnen Spaltrohren "Coil 1", "Coil 2" sind dabei als Punkte in den Diagrammen eingetragen, während die durchgezogene Linie den Mittelwert über beide Spaltrohre darstellt. C2H4, C3H6, BUTAD (**1,3-Butadien C4H6**) sowie NBUTA (**N-Butan C4H10)** sind sogenannte "high value carbons" und C2H6 sowie C3H8 sind wichtige Freiheitsgrade für die Optimierung. Es fallen noch andere Komponenten an, von denen üblicherweise z.B. auch IBUTA (**Iso-Butan C4H10**) und CH4 bei der Spaltgasanalyse gemessen werden. Alle gemessenen Komponenten können zur Bestimmung der Fehler verwendet werden.

Ausgehend von der in Fig. 2 und Fig. 3 dargestellten Situation wird nun angenommen, die Eingangsgrößen seien mit einem systematischen Fehler versehen, den das Korrekturverfahren erkennen und korrigieren soll, d.h. es soll ausgehend von den in Fig. 3 dargestellten Zusammensetzungen und einem Satz von fehlerbehafteten Eingangsgrößen ermittelt werden, welchen Wert die tatsächlichen Eingangsgrößen hatten. In der Praxis sind die entsprechenden Fehler pro Spaltrohr unterschiedlich. Es werden hier daher zwei Fälle betrachtet, um die Unterschiede zu zeigen. Im ersten Fall (Fall A) sind die Fehler bei allen Spaltrohren identisch. Dieser Fall ist insofern einfacher, als die Spaltgaszusammensetzung nur im Mittel über beide Spaltrohre bekannt ist, was jedoch bei identischen Fehlern - abgesehen vom Rauschen - identisch der Spaltgaszusammensetzung in jedem einzelnen Spaltrohr ist. Im zweiten Fall (Fall B) sind die Fehler daher unterschiedlich.

Im Fall A wurden folgende Fehler angenommen:

$$\Delta COT \quad = +5 \ ^\circ C;$$

$$\Delta DS \quad = -0.03;$$

$$\Delta FLOWR = -75 \ kg/h/coil;$$

$$\Delta COP \quad = +0.1 \ bar;$$

und im Fall B wurden folgende Fehler, jeweils pro Spaltrohr angenommen:

$$\Delta COT \quad = +5 \ ^\circ C \ bzw. \ -5 \ ^\circ C;$$

$$\Delta DS \quad = -0,03 \ bzw. \ +0,03;$$

$$\Delta FLOWR = -75 \ kg/h/coil \ (für \ beide \ Spaltrohre);$$

$$\Delta COP \quad = +0.1 \ bar \ (für \ beide \ Spaltrohre);$$

Der Vergleich umfasst drei verschiedene Methoden, deren Ziel es jeweils ist, anhand der gemessenen Spaltgaszusammensetzungen (vgl. Punkte bzw. Linien in Fig. 3 a)-f)) die Korrekturwerte der Eingangsgrößen so zu bestimmen, dass die den Eingangsgrößen zugeordneten Fehler damit jeweils genau korrigiert werden. Bei allen drei Methoden wird die Summe der Fehlerquadrate zwischen der Vorhersage, d.h. der auf Basis der fehlerhaften Eingangsgrößen erwarteten Spaltgaszusammensetzung, und der Messung der Spaltgaskomponenten minimiert. Die Messung der Spaltgaskomponenten entspricht den oben beschriebenen Zusammensetzungen bei den 300 Betriebspunkten (vgl. Fig. 3 a)-f)).

Die erste Methode entspricht der gängigen Praxis und repräsentiert den Stand der Technik. Hierbei wird das Verhältnis aus C3H6 (Propen) zu C2H4 (Ethen) gebildet, der so genannte "P/E"-Wert, und jeweils die Summe der Fehlerquadrate zwischen Messung und Vorhersage minimiert. Aufgrund der hohen Ungenauigkeit wird nur die COT-Korrektur bestimmt. Die anderen Eingangsgrößen lassen sich nicht sinnvoll korrigieren, da die Korrektur effektiv nur einen Freiheitsgrad berücksichtigt.

Als zweite Methode wird ein trivialer Ansatz verfolgt, bei dem die Anzahl der Freiheitsgrade der Anzahl der gemessenen Spaltgaskomponenten entspricht. Hierbei wird die Differenz aus allen gemessenen Spaltgaskomponenten im Vergleich zu den berechneten im Sinne der Minimierung der Fehlerquadrate minimiert. In diesem Fall geht man davon aus, dass die Eingangsgrößen und die Produktzusammensetzung laut Messung und Vorhersage bekannt ist. Die Vorhersage für die Produktzusammensetzung weicht von der Messung ab und man bildet die Summe der Fehlerquadrate. Nun werden bei den bekannten Eingangsgrößen Korrekturen addiert und mittels SPYRO die Produktzusammensetzung simuliert, um den oben berechneten Summenfehler zu minimieren.

Die dritte Methode umfasst schließlich ein Ausführungsbeispiel des erfindungsgemäßen Verfahrens. Hierbei wird mit Hilfe der Maximierung der Sensitivitäten für jeweils eine Eingangsgröße eine virtuelle Komponente berechnet, mit deren Hilfe anschließend der Fehler bzw. der Korrekturwert für die zugehörige Eingangsgröße ermittelt wird. Um die virtuellen Komponenten vorab zu bestimmen, kann beispielsweise für einen Einsatz (z.B. Naphtha) und für eine Spaltrohrgeometrie (z.B. B9601) die Spaltgaszusammensetzung für viele Variationen der Eingangsgrößen mittels eines Modells des Verarbeitungsprozesses (z.B. SPYRO) ein Kennfeld berechnet werden, welches jeder möglichen Eingangsgrößenkombination innerhalb eines erlaubten Betriebsfensters die sich jeweils einstellende Spaltgaszusammensetzung zuordnet. Dieses Kennfeld dient als Datenbasis zur Bestimmung der virtuellen Komponenten.

Mithilfe des Kennfeldes und wie weiter oben beschrieben, kann für jede Eingangsgröße anschließend eine virtuelle Komponente ermittelt werden, die (fast) ausschließlich von der jeweiligen Eingangsgröße abhängt. Als Grundfunktionen zur Berechnung der virtuellen Komponenten werden für dieses Beispiel potenzierte Verhältnisse aus den Spaltgaskomponenten verwendet. Verhältnisse deshalb, weil dann einige Probleme die sich bei der Spaltgasmessung in der Praxis ergeben (z.B. 100%-Referenz ist unbekannt) keine Rolle spielen. Es werden alle messbaren Spaltgaskomponenten, d.h. die Verhältnisse aller möglichen Kombinationen, verwendet (wobei dies nicht unbedingt erforderlich ist; wenn man weiß, dass einige z.B. aufgrund verhältnismäßig großer Messungenauigkeiten problematisch sind, wird man die nicht verwenden) und die Exponenten so gewählt, dass die zur Erstellung des Kennfeldes verwendeten Eingangsgrößen mit minimaler Abweichung im Sinne der Summe der Fehlerquadrate angenähert werden können. D.h. man rechnet mittels Regression eine virtuelle Komponente aus, die einer gewählten Eingangsgröße entsprechen soll und passt iterativ die Exponenten der Grundfunktionen an, bis die Abweichung zwischen Vorhersage der Eingangsgröße mittels Regression und dem zur Erstellung der Kennfelder verwendeten Verlauf der Eingangsgröße minimal wird. Das Grundprinzip für die Anpassung der Grundfunktionen wurde bereits im Zusammenhang mit der PLS Regression beschrieben: man kann mit sehr vielen Grundfunktionen starten (und sich die Iteration sparen), oder man startet mit weniger Grundfunktionen und verwendet z.B. die Exponenten (oder andere nicht-lineare Parameter) als Freiheitsgrad um den mittleren quadratischen Fehler (Mean Squared Error) der Regression zu minimieren, bzw. gibt es fertige Regressionsverfahren (z.B. Stepwise Regression), die einem diese Arbeit abnehmen.

Die resultierende virtuelle Komponente ist eine Linearkombination der Grundfunktionen und ist somit ausschließlich eine Funktion der gemessenen Spaltgaskomponenten. Eine virtuelle Komponente entspricht jeweils einer Eingangsgröße, d.h. es wird für jede der (vier) zu korrigierenden Eingangsgrößen auf diese Weise eine virtuelle Komponente ermittelt.

Anhand der virtuellen Komponenten kann nun mittels der gemessenen Spaltgaszusammensetzung und Berechnung der daraus folgenden virtuellen Komponenten auf die geschätzten Eingangsgrößen (d.h. eine Schätzung der wahren Eingangsgrößen) zurück gerechnet werden. Die Differenz aus den geschätzten Eingangsgrößen und den gemessenen Eingangsgrößen ist der Fehler bzw. die nötige Korrektur der Eingangsgrößen (vgl. Gleichung (6)).

Die drei Methoden wurden auf beide Fehlerfälle A und B angewandt, wobei folgende Abweichungen erkannt wurden (die entsprechenden Korrekturen hätten jeweils das umgekehrte Vorzeichen):

| Fall A | COT [°C] | DS [1] | FLOWR [kg/h/ coil] | COP [bar] |
|---|---|---|---|---|
| Tatsächlicher (aufgegebener) Fehler | +5 | -0.03 | -75 | 0.1 |
| Mittels Spaltschärfe P/E | 8.532 | - | - | - |
| Unter Verwendung aller gemessenen Spaltgaskomponenten | 5.754 | -0.0098 | -0.6 | 0.1573 |
| Mittels Maximierung der Sensitivitäten (Erfindung) | 5.000 | -0.0310 | -75.7 | 0.0977 |

| Fall B | COT [°C] | DS [1] | FLOWR [kg/h/ coil] | COP [bar] |
|---|---|---|---|---|
| Tatsächlicher (aufgegebener) Fehler | +5/-5 | - 0.03/0. 03 | -75/-75 | +0.1/+0. 1 |
| Arithmetischer Mittelwert der Fehler | 0 | 0 | -75 | 0.1 |
| Mittels Spaltschärfe P/E | -2.695 | - | - | - |
| Unter Verwendung aller gemessenen Spaltgaskomponenten | -1.160 | 0.0903 | -0.2 | -0.0541 |
| Mittels Maximierung der Sensitivitäten (Erfindung) | 0.000 | 0.0009 | -75.7 | 0.0979 |

Im Fall B konnte naturgemäß nur eine Abweichung ermittelt werden, weil nur die mittlere Spaltgaszusammensetzung (d.h. im Mittelwert über beide Spaltrohre) gemessen wurde bzw. bekannt war. Die mit Abstand beste Schätzung der Abweichungen gelingt in beiden Fällen A und B mittels des erfindungsgemäßen Verfahrens und der damit erzielten Maximierung der Sensitivitäten.

Abschließend wurde für mehrere vom Ausgangszustand abweichende Validierungsbetriebspunkte die Vorhersage basierend auf den korrigierten Eingangsgrößen berechnet und wieder die unterschiedlichen Methoden verglichen. Zur Validierung werden sechs willkürlich gewählte, aber das Betriebsfenster abdeckende Validierungszustände, wiederum inkl. Prozessrauschen, geschaffen. Diese Validierungszustände können z.B. Vorgaben seitens eines Optimierungssystems sein.

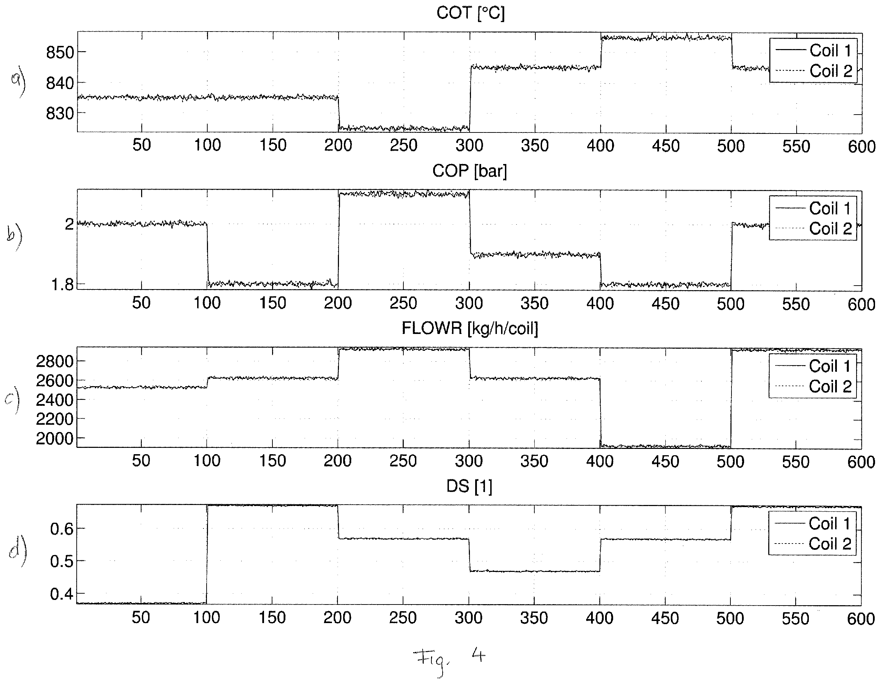

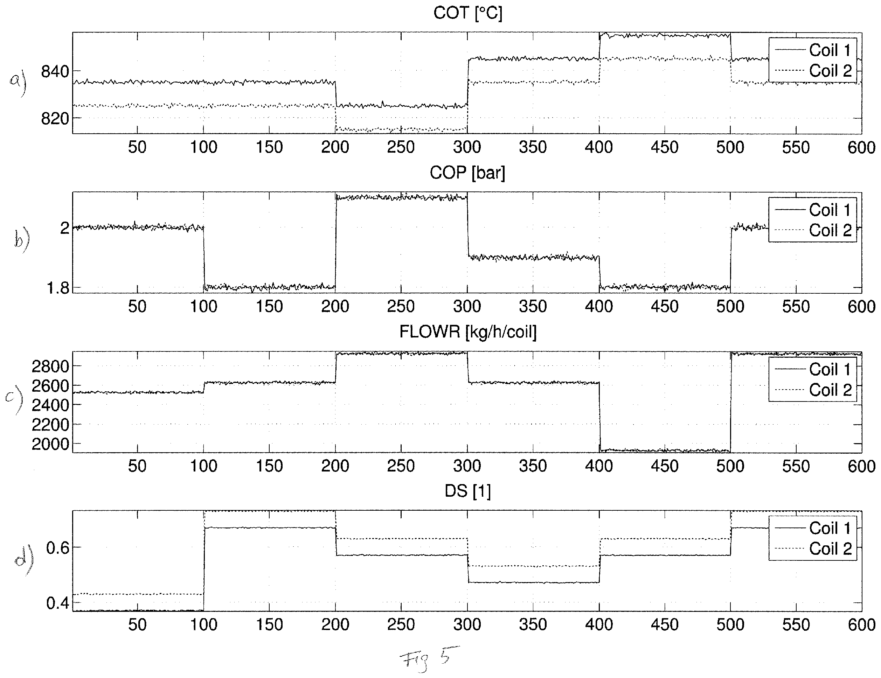

Die Fig. 4 und 5 zeigen jeweils vier Diagramme a)-d) der jeweils insgesamt 600 Validierungsbetriebspunkte, welche jeweils auf sechs verschiedene Fehlersituationen entsprechend den Validierungszuständen aufgeteilt sind, mit einem entsprechenden Prozessrauschen. Die beiden in jedem Diagramm a)-d) dargestellten Linien "Coil 1", "Coil 2" entsprechend dabei jeweils einem Spaltrohr des Ofens, d.h. die in Fig. 4 - abgesehen vom Prozessrauschen - überlagerten Linien entsprechend Fall A, d.h. die Fehler sind für beide Spaltrohre identisch. In Fig. 5 sind auch die systematischen Fehler spaltrohrabhängig, d.h. die mittlere COT gemäß Fig. 5 a) und der mittlere DS gemäß Fig. 5 d) sind in den beiden Spaltrohren verschieden.

Mit Hilfe der zuvor berechneten Korrekturen können die Eingangsgrößen korrigiert und infolge die sich für jede Methode ergebende Spaltgaszusammensetzung berechnet werden. Im besten Fall ist die Differenz zwischen der mittels der korrigierten Eingangsgrößen vorhergesagten Spaltgaszusammensetzung und der gemessenen Spaltgaszusammensetzung gleich 0. Je größer die Abweichung ist, desto schlechter das Ergebnis. Optimierungssysteme nützen Verschiebungen im einstelligen Prozentbereich aus. Wird daher eine Komponente um ein paar Prozent falsch vorhergesagt, ist somit das gesamte Optimum fragwürdig. Abweichungen in der Größenordnung von > 2-3 % führen dazu, dass eine Optimierung der Spaltgaszusammensetzung einem Glücksspiel gleicht.

Die Recyclingströme C2H6 und C3H8 mögen zwar keinen großen direkten materiellen Wert haben, sind aber wichtige Einsätze und somit wichtige Freiheitsgrade. D.h. es sind nicht nur die "high value carbons" (C2H4, C3H6, BUTAD usw.) bei der Vorhersage von Bedeutung. Für die einzelnen Spaltgaskomponenten K ergeben sich die relativen Vorhersagefehler (in %) zu:

$$\frac{K_{\text{Vorhersage}} - K_{\text{Messung}}}{K_{Messung}} \cdot 100 \qquad (7)$$

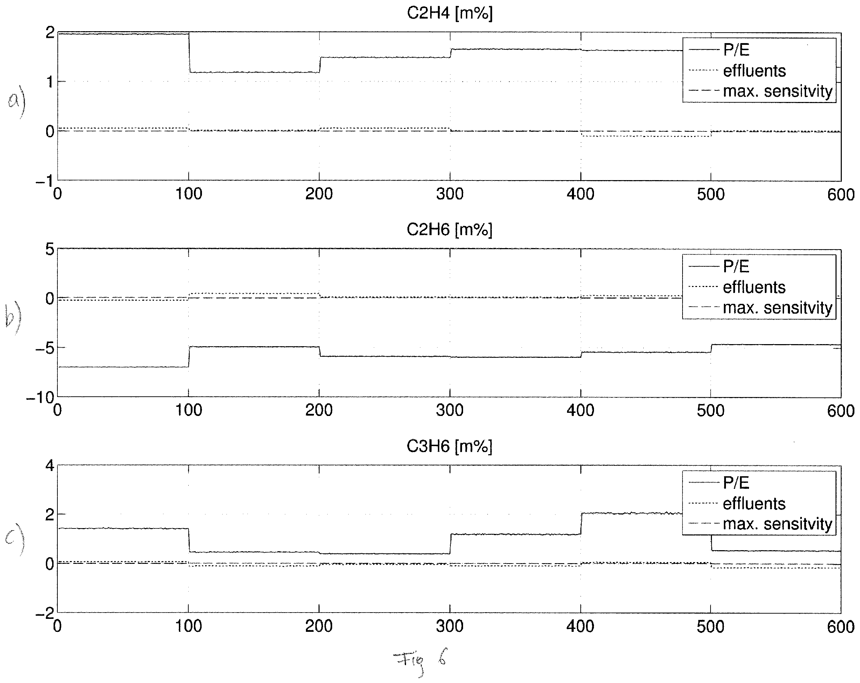

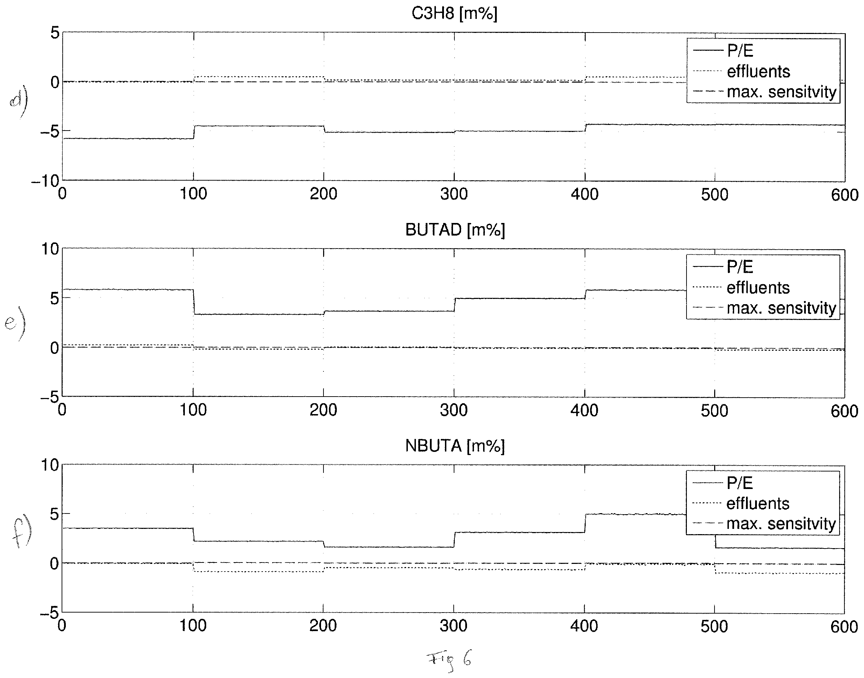

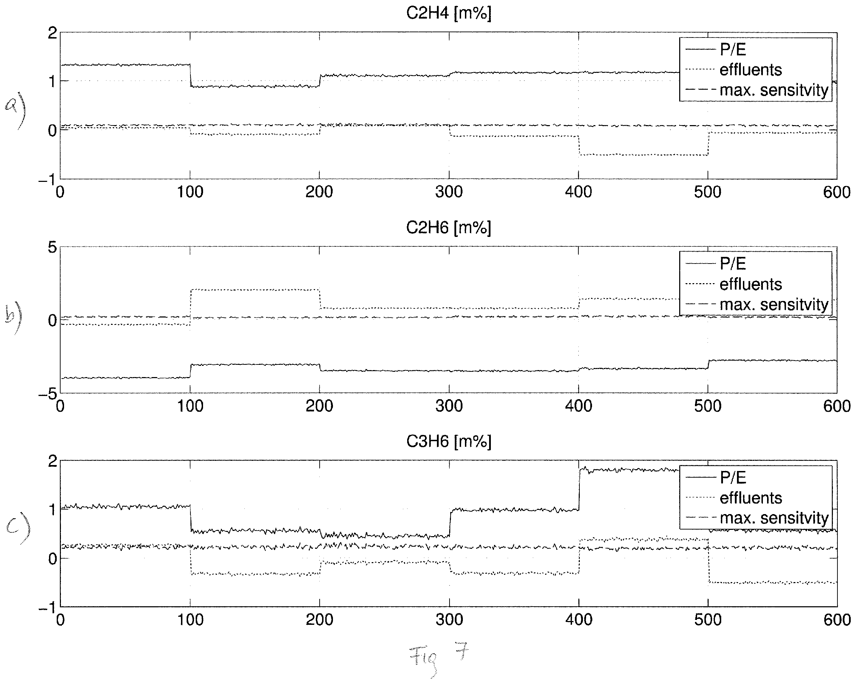

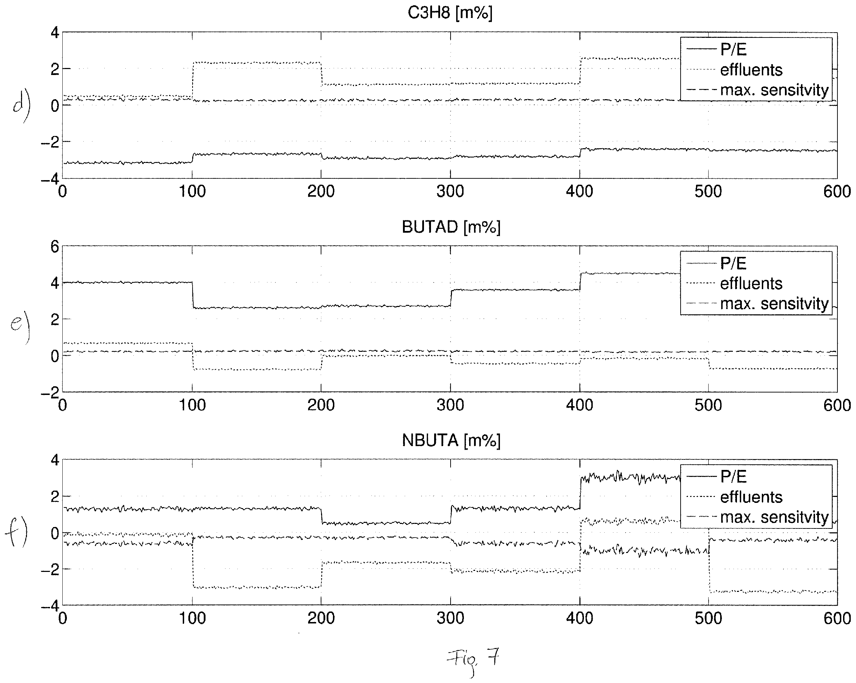

Die sich aus den Validierungsbetriebspunkten ergebenden relativen Vorhersagefehler sind in Fig. 6 und 7 dargestellt, wobei Fig. 6 dem Fall A mit identischen Fehlern in beiden Spaltrohren (vgl. Fig. 4) und Fig. 7 dem Fall B mit unabhängigen Fehlern (vgl. Fig. 5) entspricht. Jedes der Diagramme a)-f) in Fig. 6 bzw. 7 entspricht dabei einer Spaltgaskomponente und die drei Linien "P/E", "effluents", "max. sensitvity" korrespondieren zu den drei hier verglichenen Methoden und zeigen deren relativen Vorhersagefehler an dem jeweiligen Betriebspunkt an.

Die dritte Methode ("Fehlerkorrektur mittels Maximierung der Sensitivität" bzw. "max.sensitvity") auf Basis des erfindungsgemäßen Verfahrens ist wiederum ungeschlagen. Mit dieser Methode kann die Spaltgaszusammensetzung zur Optimierung genutzt werden. Die Vorhersagefehler sind nahezu 0.

Die Vorhersagefehler nach der zweiten Methode ("Fehlerkorrektur mittels Verw. aller Spaltgaskomponenten" bzw. "effluents") sind deutlich schlechter als bei der dritten Methode. Je nach Betriebspunkt können sich signifikante Abweichungen ergeben, siehe BUTAD (vgl. Fig. 6 e) und Fig. 7 e)) und C3H6 (vgl. Fig. 6 c) und Fig. 7 c)) (aber auch C2H6 und C3H8 machen Probleme). Nachdem a priori unbekannt ist, in welchem Betriebspunkt die Vorhersagen stimmen und in welchem nicht, ist eine auf der Optimierung der Spaltgaszusammensetzung basierende Deckungsbeitragssteigerung kaum möglich - bzw. muss man mit einem großen Deckungsbeitragsverlust rechnen.

Die Vorhersagefehler mittels der ersten Methode ("Fehlerkorrektur mittels P/E" bzw. kurz "P/E") liegen wieder deutlich im einstelligen Prozentbereich. Eine auf der Optimierung der Spaltgaszusammensetzung basierende Deckungsbeitragssteigerung ist nicht sinnvoll möglich.

Abschließend soll anhand eines konkreten Beispiels die Anwendung des erfindungsgemäßen Verfahrens, insbesondere die Ermittlung einer virtuellen Komponente, noch weiter erläutert werden.

Dabei wird zunächst, wie bereits mehrfach ausgeführt, eine Reihe von "Grundfunktionen" festgelegt, die alle eine Funktion der gemessenen Spaltgaskomponenten sind, beispielsweise

$$f_1 = \left(\frac{C2H4}{C3H6}\right)^{a1}, \quad f_2 = \left(\frac{CH4}{C2H6}\right)^{a2}, \quad f_3 = \left(\frac{CH4}{C3H8}\right)^{a3}, \quad \text{usw.} \qquad (8)$$

Diese Grundfunktionen werden zu einer Matrix zusammengefasst, wobei jede Spalte einer Grundfunktion und jede Zeile einem Betriebspunkt entspricht; jeder Betriebspunkt entspricht einer bestimmten Kombination der Eingangsgrößen. Die erste Spalte muss einer beliebigen Konstante entsprechen, um bei der Regression den "0-Punkt" zu definieren.

$$X = \begin{bmatrix} 1, & f_1, & f_2, & f_3, & f_4, & \cdots \end{bmatrix} \qquad (9)$$

Die zugehörige COT bzw. der zugehörige Parameter wird zu einem Response-Spaltenvektor entsprechend den Werten bei den verschiedenen Betriebspunkten zusammengefasst:

$$y = \begin{bmatrix} COT \end{bmatrix} \qquad (10)$$

Mittels Regression wird nun bestimmt, wie die Gewichtungen (lineare Superposition) der einzelnen Grundfunktionen aussehen müssen, um eine optimale Näherung der COT in Abhängigkeit der Grundfunktionen zu ermöglichen. Zusätzlich kann man die Exponenten a iterativ dahingehend optimieren, dass die Abweichung zwischen der geschätzten COT und dem wahren Wert Mathematisch ausgedrückt wird im Zuge der Regression ein Spaltenvektor b so bestimmt, dass die Summe der quadrierten Fehler $y_{res}$ minimiert wird (lineare Regression).

$$y = X \cdot b + y_{res} \ , \qquad (11)$$

wobei die Einträge des Vektors b angeben, wie die einzelnen Grundfunktionen gewichtet aufsummiert werden müssen, um die gesuchte virtuelle Komponente zu erhalten. Zur Bestimmung der COT-Korrektur muss nur noch folgende Differenz berechnet werden:

$$COT_{Korrektur} = X_{gemessen} \cdot b - X_{erwartet} \cdot b \ , \qquad (12)$$

$X_{gemessen}$ wird genauso berechnet wie $X_{erwartet}$, wobei anstelle der auf Basis der - im Allgemeinen fehlerhaften - Eingangsgrößen berechneten Spaltgaskomponenten die gemessenen eingesetzt werden.

Im Fall der COT-Korrektur ergibt sich für einen hier beispielhaft angenommenen Einsatz und den Ofen des Typs B9601 die Matrix X (hier durch die Grundfunktionen mit den aufgefundenen Exponenten ausgedrückt) bzw. der Vektor b z.B. wie folgt (zur besseren Lesbarkeit wurden Kommas hier gemäß der englischen Schreibweise als Punkt angegeben):

```
X = [1, (CH4/C2H4)^-0.16617, (CH4/C2H4)^-0.36981, (CH4/C2H6)^-
0.16617, (CH4/C2H6)^-0.36981, (CH4/C3H6)^-0.16617, (CH4/C3H6)^-
0.36981, (CH4/C3H8)^-0.16617, (CH4/C3H8)^-0.36981, (CH4/BUTAD)^-
0.16617, (CH4/BUTAD)^-0.36981, (CH4/NBUTA)^-0.16617,
(CH4/NBUTA)^-0.36981, (CH4/IBUTA)^-0.16617, (CH4/IBUTA)^-
0.36981, (C2H4/C2H6)^-0.16617, (C2H4/C2H6)^-0.36981,
(C2H4/C3H6)^-0.16617, (C2H4/C3H6)^-0.36981, (C2H4/C3H8)^-
0.16617, (C2H4/C3H8)^-0.36981, (C2H4/BUTAD)^-0.16617,
(C2H4/BUTAD)^-0.36981, (C2H4/NBUTA)^-0.16617, (C2H4/NBUTA)^-
0.36981, (C2H4/IBUTA)^-0.16617, (C2H4/IBUTA)^-0.36981,
(C2H6/C3H6)^-0.16617, (C2H6/C3H6)^-0.36981, (C2H6/C3H8)^-
0.16617, (C2H6/C3H8)^-0.36981, (C2H6/BUTAD)^-0.16617,
(C2H6/BUTAD)^-0.36981, (C2H6/NBUTA)^-0.16617, (C2H6/NBUTA)^-
0.36981, (C2H6/IBUTA)^-0.16617, (C2H6/IBUTA)^-0.36981,
(C3H6/C3H8)^-0.16617, (C3H6/C3H8)^-0.36981, (C3H6/BUTAD)^-
0.16617, (C3H6/BUTAD)^-0.36981, (C3H6/NBUTA)^-0.16617,
(C3H6/NBUTA)^-0.36981, (C3H6/IBUTA)^-0.16617, (C3H6/IBUTA)^-
0.36981, (C3H8/BUTAD)^-0.16617, (C3H8/BUTAD)^-0.36981,
(C3H8/NBUTA)^-0.16617, (C3H8/NBUTA)^-0.36981, (C3H8/IBUTA)^-
0.16617, (C3H8/IBUTA)^-0.36981, (BUTAD/NBUTA)^-0.16617,
(BUTAD/NBUTA)^-0.36981, (BUTAD/IBUTA)^-0.16617, (BUTAD/IBUTA)^-
0.36981, (NBUTA/IBUTA)^-0.16617, (NBUTA/IBUTA)^-0.36981];


b = 1.0e+05 * [-1.9727, -1.8331, 0.8225, 0.5513, 0.0031, -
2.0568, 0.8497, 3.9290, -1.0625, -0.5895, -0.2377, 0.1097, -
0.2639, -3.2815, 0.7517, 0.6523, -0.4119, 1.7483, -0.6788, -
2.9678, 0.9010, -0.9186, 0.5984, -0.0817, 0.0425, 2.7961, -
0.6345, 1.5415, -0.2819, -0.8762, 0.3507, -0.5641, 0.1591, -
0.9537, 0.3191, 1.2039, -0.4573, 3.9483, -1.7377, -1.6233,
0.6138, -1.2763, 1.0388, 2.0271, -1.0116, 1.1303, -0.1244,
1.4036, -0.3317, -1.2293, 0.5085, 0.0247, -0.0581, -1.1911,
0.3464, 0.4801, -0.2098].
```

Die hier genauer dargelegte Bestimmung der COT-Korrektur mittels des SPYRO Modells dient nur als Beispiel. Die Methodik kann in analoger Weise und mit vergleichbaren Vorteilen überall angewendet werden, wo ähnliche Probleme (z.B. Parameteranpassung von Modellen) zu lösen sind.

Der obige Vergleich, d.h. konkret die oben angegebenen Tabellen sowie Fig. 6 und 7, zeigt eindeutig, dass eine Parameteranpassung gemäß dem Stand der Technik, die nur auf P/E beruht, keine zufriedenstellenden Ergebnisse liefert. Die Spaltgaszusammensetzung kann dabei nicht bzw. nur unzureichend zur Optimierung genutzt werden, da die Vorhersagen des Optimierungssystems viel zu ungenau sind.

Eine Parameteranpassung die alle gemessenen Spaltgaskomponenten verwendet, aber diese lediglich in einen linearen Zusammenhang setzt, birgt jedoch folgende Probleme: wenn nicht die Verhältnisse von Spaltgaskomponenten zur Berechnung der quadrierten Fehler verwendet werden, sondern direkt die gemessenen Werte, d.h. es wird nicht z.B. P/E verwendet sondern P und E usw., ergibt sich das Problem, dass dies in der Praxis aufgrund analysetechnischer Einschränkungen nicht funktioniert; z.B. fehlt aufgrund der Probennahme die 100% Referenz, welche bei Verwendung von Verhältnissen keine Rolle spielt. Selbst wenn die Spaltgasanalyse diese Probleme nicht hätte, ist die Vorhersage nur in einigen Betriebspunkten ausreichend genau, in anderen jedoch nicht. Da a priori nicht bekannt ist, ob die Vorhersage zufällig stimmt oder nicht, lässt sich damit auch kaum die Spaltgaszusammensetzung zur Erhöhung des Deckungsbeitrages nützen. Daraus folgt, dass eine Parameteranpassung die - wie hier gezeigt - alle gemessenen Spaltgaskomponenten verwendet, ebenso wenig zur Deckungsbeitragssteigerung durch Optimierung der Spaltgaskomponenten geeignet ist.

Erst eine Parameteranpassung, d.h. eine Korrektur der Eingangsgrößen, basierend auf der Maximierung der Sensitivitäten hingegen, und unter Verwendung des erfindungsgemäßen Verfahrens, ermöglicht unabhängig vom Betriebspunkt beste Ergebnisse. Erst in Kombination mit diesem Verfahren lässt sich eine optimale Wahl der Spaltgaszusammensetzung zur Erhöhung des Deckungsbeitrages effizient nutzen.

**Patentansprüche**

1. Verfahren zur Korrektur einer Messung einer Eingangsgröße (COT; COP; FLOWR; DS) eines chemischen Verarbeitungsprozesses zur Herstellung eines aus mehreren Produktkomponenten (C2H4, C2H6, C3H6, C3H8, BUTAD, NBUTA) zusammengesetzten Produkts, umfassend die Schritte, dass während des Verarbeitungsprozesses

   - die Eingangsgröße (COT; COP; FLOWR; DS) gemessen wird;
   - die Anteile mehrerer Produktkomponenten (C2H4, C2H6, C3H6, C3H8, BUTAD, NBUTA) am Produkt gemessen werden;
   - ein Korrekturwert der Eingangsgröße (COT; COP; FLOWR; DS) anhand der gemessenen Anteile ermittelt und für zukünftige Messungen gespeichert wird,
   **dadurch gekennzeichnet, dass**
   - die Anteile zumindest dreier Produktkomponenten (C2H4, C2H6, C3H6, C3H8, BUTAD, NBUTA) gemessen werden,
   und dass zur Ermittlung des Korrekturwerts
   - ein aktueller Anteil einer virtuellen Komponente des Produkts anhand einer nicht-linearen Abbildung der zumindest drei gemessenen Anteile ermittelt wird, wobei die nicht-lineare Abbildung eine Linearkombination von zumindest zwei nichtlinearen Grundfunktionen der gemessenen Anteile ist, wobei jeder Grundfunktion durch multivariable lineare Regression ausgehend von einem Vorhersagemodell des chemischen Verarbeitungsprozesses ein Koeffizient zur Gewichtung der Grundfunktion zugewiesen wird, sodass zumindest innerhalb eines Betriebsfensters mehrerer Eingangsgrößen (COT, COP, FLOWR, DS) der Einfluss der übrigen Eingangsgrößen auf die virtuelle Komponente kleiner ist als jener der gemessenen Eingangsgröße (COT; COP; FLOWR; DS); und
   - der Korrekturwert aus dem aktuellen Anteil der virtuellen Komponente und der gemessenen Eingangsgröße (COT; COP; FLOWR; DS) ermittelt wird.

2. Verfahren nach Anspruch 1, **dadurch gekennzeichnet, dass** zur Ermittlung des Korrekturwerts

   - ein erwarteter Anteil der virtuellen Komponente ausgehend von der gemessenen Eingangsgröße (COT; COP; FLOWR; DS) und einem Vorhersagemodell des Verarbeitungsprozesses ermittelt wird; und
   - der Korrekturwert aus der Abweichung des aktuellen Anteils der virtuellen Komponente von dem erwarteten Anteil der virtuellen Komponente ermittelt wird.

3. Verfahren nach Anspruch 1 oder 2, **dadurch gekennzeichnet, dass** zumindest zwei Eingangsgrößen (COT, COP, FLOWR, DS) gemessen werden und jeweils ein Korrekturwert ermittelt wird, wobei jeder Eingangsgröße (COT, COP, FLOWR, DS) eine eigene virtuelle Komponente entsprechend einer individuellen nichtlinearen Abbildung zugeordnet ist.

4. Verfahren nach einem der Ansprüche 1 bis 3, **dadurch gekennzeichnet, dass** eine der Eingangsgröße (COT; COP; FLOWR; DS) entsprechende Stellgröße des Verarbeitungsprozesses geregelt wird, wobei eine Regelabweichung der Stellgröße in Abhängigkeit von dem ermittelten und gespeicherten Korrekturwert der Eingangsgröße (COT; COP; FLOWR; DS) ermittelt wird.

**5.** Verfahren nach einem der Ansprüche 1 bis 4, **dadurch gekennzeichnet, dass** die virtuelle(n) Komponente(n), zumindest innerhalb eines Betriebsfensters mehrerer Eingangsgrößen (COT, COP, FLOWR, DS) des Vorhersagemodells, im Wesentlichen nur von einer einzigen Eingangsgröße abhängig und dieser zugeordnet ist bzw. sind.

**6.** Verfahren nach einem der Ansprüche 1 bis 5, **dadurch gekennzeichnet, dass** zumindest zwei Verhältnisse der Anteile der zumindest drei Produktkomponenten (C2H4, C2H6, C3H6, C3H8, BUTAD, NBUTA) in die virtuelle(n) Komponente(n) eingehen.

**7.** Verfahren nach einem der Ansprüche 1 bis 6, **dadurch gekennzeichnet, dass** die gemessenen Anteile mit einem jeweils zugeordneten reellen Exponenten in die virtuelle(n) Komponente(n) eingehen.

**8.** Verfahren nach einem der Ansprüche 1 bis 7, **dadurch gekennzeichnet, dass** die Koeffizienten der nicht-linearen Abbildung durch multivariable Regression, ausgehend von einem Vorhersagemodell des chemischen Verarbeitungsprozesses zur Approximation des Vorhersagemodells innerhalb eines vorgesehenen Betriebsfensters des Verarbeitungsprozesses bestimmt werden.

**9.** Verfahren nach einem der Ansprüche 1 bis 8, **dadurch gekennzeichnet, dass** die Koeffizienten aus einem Kennfeld der erwarteten Produktzusammensetzungen aller möglichen oder zumindest aller vorausgesehenen Eingangsgrößenkonfigurationen innerhalb des Betriebsfensters des Verarbeitungsprozesses abgeleitet werden, wobei das Kennfeld aus einem Vorhersagemodell des Verarbeitungsprozesses generiert wird.

**10.** Verfahren nach einem der Ansprüche 1 bis 9, **dadurch gekennzeichnet, dass** bei der Ableitung der Koeffizienten alle im Rahmen des Verarbeitungsprozesses gemessenen bzw. messbaren Anteile von Produktkomponenten berücksichtigt werden.

**11.** Verfahren nach einem der Ansprüche 1 bis 10, **dadurch gekennzeichnet, dass** bei der Ableitung der Koeffizienten mit den gemessenen Anteilen der Produktkomponenten (C2H4, C2H6, C3H6, C3H8, BUTAD, NBUTA) assoziierte Messfehler berücksichtigt werden, indem vorzugsweise genauer messbaren Anteilen ein höheres Gewicht gegeben wird als weniger genau messbaren.

**12.** Verfahren nach einem der Ansprüche 1 bis 11, **dadurch gekennzeichnet, dass** der chemische Verarbeitungsprozess ein Dampfspaltungsprozess ist, wobei das Produkt ein Spaltgas ist und die Produktkomponenten Spaltgaskomponenten sind, insbesondere im Spaltgas enthaltene Kohlenwasserstoffe.

**13.** Verfahren nach Anspruch 12, **dadurch gekennzeichnet, dass** die Eingangsgröße(n) (COT; COP; FLOWR; DS) eine Spaltgastemperatur (COT) an einem Spaltrohr-Austritt bzw. an einem Ofenaustritt, ein Spaltgasdruck (COP) am Spaltrohr-Austritt bzw. am Ofenaustritt, eine Einsatzmenge (FLOWR) an zu spaltenden Kohlenwasserstoffen an einem Spaltrohr-Eintritt, eine Einsatzzusammensetzung und/oder ein Verhältnis (DS) eines Prozessdampf-Volumens zur Einsatzmenge ist bzw. umfassen.

**14.** Vorrichtung zur Korrektur einer Messung einer Eingangsgröße (COT; COP; FLOWR; DS) eines chemischen Verarbeitungsprozesses zur Herstellung eines aus mehreren Produktkomponenten (C2H4, C2H6, C3H6, C3H8, BUTAD, NBUTA) zusammengesetzten Produkts gemäß dem Verfahren nach einem der Ansprüche 1 bis 13, mit Messmitteln (12) zur Messung der Eingangsgröße (COT; COP; FLOWR; DS) und zur Messung der Anteile mehrerer Produktkomponenten (C2H4, C2H6, C3H6, C3H8, BUTAD, NBUTA) am Produkt, welche Messmittel (12) mit Rechenmitteln (13) verbunden sind, wobei die Rechenmittel (13) eingerichtet sind, einen Korrekturwert der Eingangsgröße (COT; COP; FLOWR; DS) anhand der gemessenen Anteile zu ermitteln, **dadurch gekennzeichnet, dass** die Messmittel (12) eingerichtet sind, die jeweiligen Anteile von zumindest drei Produktkomponenten (C2H4, C2H6, C3H6, C3H8, BUTAD, NBUTA) zu messen, und dass die Rechenmittel (13) eingerichtet sind, den Korrekturwert aus einem Messwert der Eingangsgröße (COT; COP; FLOWR; DS) und einem aktuellen Anteil einer virtuellen Komponente, welche einer nicht-linearen Abbildung der zumindest drei gemessenen Anteile entspricht, zu berechnen, wobei die nicht-lineare Abbildung eine Linearkombination von nichtlinearen Grundfunktionen der gemessenen Anteile ist, wobei jeder Grundfunktion durch multivariable lineare Regression ausgehend von einem Vorhersagemodell des chemischen Verarbeitungsprozesses ein Koeffizient zur Gewichtung der Grundfunktion zugewiesen wird, sodass zumindest innerhalb eines Betriebsfensters mehrerer Eingangsgrößen (COT, COP, FLOWR, DS) der Einfluss der übrigen Eingangsgrößen auf die virtuelle Komponente kleiner ist als jener der gemessenen Eingangsgröße (COT; COP; FLOWR; DS).

**Claims**

1. A method of correcting a measurement of an input parameter (COT; COP; FLOWR; DS) of a chemical process for producing a product composed of a plurality of product components (C2H4, C2H6, C3H6, C4H8, BUTAD, NBUTA) including the steps that during the process,

   - the input parameter (COT; COP, FLOWR; DS) is measured;
   - the proportions of a plurality of product components (C2H4, C2H6, C3H6, C3H8, BUTAD, NBUTA) in the product are measured;
   - a correction value of the input parameter (COT, COP, FLOWR; DS) is determined with the aid of the measured proportions and stored for future measurements **characterised in that**,
   - the proportions of at least three product components (C2H4, C2H6, C3H6, C3H8, BUTAD, NBUTA) are measured, and that to determine the correction value
   - a current proportion of a virtual component of the product is determined with the aid of a non-linear mapping of the at least three measured proportions, wherein the non-linear mapping is a linear combination of at least two basic functions of the measured proportions, wherein a coefficient for weighting the basic function is assigned to each basic function by multivariable linear regression starting from a predictive model of the chemical process so that at least within an operational window of a plurality of input parameters (COT, COP, FLOWR, DS) the influence of the other input parameters on the virtual component is smaller than that of the measured input parameter (COT, COP, FLOWR; DS); and
   - the correction value is determined from the current proportion of the virtual component and the measured input parameter (COT; COP; FLOWR; DS).

2. A method as claimed in claim 1, **characterised in that** to determine the correction value

   - an expected proportion of the virtual component is determined starting from the measured input parameter (COT; COP; FLOWR; DS) and a predictive model of the process; and
   - the correction value is determined from the deviation of the current proportion of the virtual component from the expected proportion of the virtual component.

3. A method as claimed in claim 1 or 2, **characterised in that** at least two input parameters (COT; COP; FLOWR; DS) are measured and in each case a correction value is determined, wherein an individual virtual component corresponding to an individual non-linear mapping is assigned to each input parameter (COT; COP; FLOWR; DS).

4. A method as claimed in one of claims 1 to 3, **characterised in that** a control variable, corresponding to the input parameter (COT; COP; FLOWR; DS), of the process is controlled, whereby a control deviation of the control variable is determined in dependence on the determined and stored correction value of the input parameter (COT; COP; FLOWR; DS).

5. A method as claimed in one of claims 1 to 4, **characterised in that** the virtual component(s), at least within an operational window of a plurality of input parameter (COT, COP, FLOWR, DS) of the predictive model, is or are dependent on a single input parameter and associated with it.

6. A method as claimed in one of claims 1 to 5, **characterised in that** at least two ratios of the proportions of at least three product components (C2H4, C2H6, C3H6, C3H8, BUTAD, NBUTA) go into the virtual component(s).

7. A method as claimed in one of claims 1 to 6, **characterised in that** the measured proportions with a respective associated real exponent go into the virtual component(s).

8. A method as claimed in one of claims 1 to 7, **characterised in that** the coefficients of the non-linear mapping are determined by multivariable regression, starting from a predictive model of the chemical process for approximating the predictive model within a provided operational window of the process.

9. A method as claimed in one of claims 1 to 8, **characterised in that** the coefficients are derived from a characteristic map of the expected product compositions of all possible or at least all foreseen input parameter configurations within the operational window of the process, wherein the characteristic map is generated from a predictive model of the process.

**10.** A method as claimed in one of claims 1 to 9, **characterised in that** all of the proportions of product components which are measured or measurable in the context of the process are taken account of in the derivation of the coefficients.

**11.** A method as claimed in one of claims 1 to 10, **characterised in that** the derivation of the coefficients with the measured proportions of the product components (C2H4, C2H6, C3H6, C3H8, BUTAD, NBUTA) associated measuring errors are taken account of **in that**, preferably, more precisely measurable proportions are given greater weight than those which are less precisely measurable.

**12.** A method as claimed in one of claims 1 to 11, **characterised in that** the chemical process is a vapour cracking process, wherein the product is a cracked gas and the product components are cracked gas components, particularly hydrocarbons contained in the cracked gas.

**13.** A method as claimed in claim 12, **characterised in that** the input parameter(s) (COT; COP; FLOWR; DS) is or include a cracked gas temperature (COT) at a cracker pipe outlet or at a furnace outlet, a cracked gas pressure (COP) at the cracker pipe outlet or at the furnace outlet, an input amount (FLOWR) of hydrocarbons to be cracked at a cracker pipe inlet, an input composition and/or a ratio (DS) of a process vapour volume to the input amount.

**14.** Apparatus for correcting a measurement of an input parameter (COT; COP; FLOWR; DS) of a chemical process for producing a product composed of a plurality of product components (C2H4, C2H6, S3H6, C3H8, BUTAD, NBUTA) in accordance with the method as claimed in one of claims 1 to 13 with measuring means (12) for measuring the input parameter (COT; COP; FLOWR; DS) and for measuring the proportions of a plurality of product components (CHH4, C2H6, C3H6, BUTAD, NBUTA) in the product, which measuring means (12) are connecting to computing means (13), wherein the computing means (13) are arranged to determine a correction value for the input parameter (COT; COP; FLOWR; DS) with the aid of the measured proportions, **characterised in that** the measuring means (12) are arranged to measure the respective proportions of at least three product components (C2H4, C2H6, C3H6, C3H8, BUTAD, NBUTA) and that the computing means (13) are arranged to calculate the correction value from a measured value of the input parameter (COT; COP; FLOWR; DS) and a current proportion of a virtual component, which corresponds to a non-linear mapping of the at least three measured proportions, wherein the non-linear mapping is a linear combination of non-linear basic functions of the measured proportions, wherein assigned to each basic function by multivariable linear regression starting from a predictive model of the chemical process there is a coefficient for weighting the basic function so that, at least within an operational window of a plurality of input parameters (COT, COP, FLOWR, DS), the influence of the other input parameters on the virtual component is smaller than that of the measured input parameter (COT; COP; FLOWR; DS).

**Revendications**

**1.** Procédé destiné à la correction d'une mesure d'une grandeur d'entrée (COT ; COP ; FLOWR ; DS) d'un processus de transformation chimique pour la fabrication d'un produit composé de plusieurs composants de produit (C2H4, C2H6, C3H6, C3H8, BUTAD, NBUTA), comprenant les étapes suivantes, pendant le processus de transformation,

- mesurer la grandeur d'entrée (COT ; COP ; FLOWR ; DS) ;
- mesurer les proportions de plusieurs composants de produit (C2H4, C2H6, C3H6, C3H8, BUTAD, NBUTA) dans le produit ;
- déterminer une valeur de correction de la grandeur d'entrée (COT ; COP ; FLOWR ; DS) à l'aide des proportions mesurées et l'enregistrer pour des mesures futures, **caractérisé en ce que** :
- les proportions d'au moins trois composants de produit (C2H4, C2H6, C3H6, C3H8, BUTAD, NBUTA) sont mesurées,

et **en ce que**, pour déterminer la valeur de correction,

- une proportion actuelle d'un composant virtuel du produit est déterminée à l'aide d'une application non linéaire des au moins trois proportions mesurées, où l'application non linéaire est une combinaison linéaire d'au moins deux fonctions de base non linéaires des proportions mesurées, où chaque fonction de base se voit attribuer un coefficient de pondération de la fonction de base par régression linéaire à plusieurs variables en partant d'un modèle prédictif du processus de transformation chimique, de telle sorte que, au moins à l'intérieur d'une fenêtre d'exploitation de plusieurs grandeurs d'entrée (COT, COP, FLOWR, DS), l'influence des grandeurs d'entrée restantes sur le composant virtuel soit inférieure à celle de la grandeur d'entrée mesurée (COT ; COP ; FLOWR ; DS) ; et
- la valeur de correction est déterminée à partir de la proportion actuelle du composant virtuel et de la grandeur d'entrée mesurée (COT ; COP ; FLOWR ; DS).

2. Procédé selon la revendication 1, **caractérisé en ce que**, pour déterminer la valeur de correction,

- une proportion escomptée du composant virtuel est déterminée en partant de la grandeur d'entrée mesurée (COT ; COP ; FLOWR ; DS) et d'un modèle prédictif du processus de transformation ; et
- la valeur de correction est déterminée à partir de la différence entre la proportion actuelle du composant virtuel et la proportion escomptée du composant virtuel.

3. Procédé selon la revendication 1 ou 2, **caractérisé en ce qu'**au moins deux grandeurs d'entrée (COT, COP, FLOWR, DS) sont mesurées et, à chaque fois, une valeur de correction est déterminée, où chaque grandeur d'entrée (COT, COP, FLOWR, DS) se voit affecter d'un propre composant virtuel conformément à une application non linéaire individuelle.

4. Procédé selon l'une des revendications 1 à 3, **caractérisé en ce qu'**une grandeur de réglage du processus de transformation correspondant à la grandeur d'entrée (COT ; COP ; FLOWR ; DS) est réglée, où une différence de réglage de la grandeur de réglage est déterminée en fonction de la valeur de correction de la grandeur d'entrée (COT ; COP ; FLOWR ; DS) déterminée et enregistrée.

5. Procédé selon l'une des revendications 1 à 4, **caractérisé en ce que** le ou les composants virtuels, au moins à l'intérieur d'une fenêtre d'exploitation de plusieurs grandeurs d'entrée (COT, COP, FLOWR, DS) du modèle prédictif, sont sensiblement dépendants seulement d'une unique grandeur d'entrée et y sont affectés.

6. Procédé selon l'une des revendications 1 à 5, **caractérisé en ce qu'**au moins deux rapports des proportions des au moins trois composants de produit (C2H4, C2H6, C3H6, C3H8, BUTAD, NBUTA) rentrent dans le ou les composants virtuels.

7. Procédé selon l'une des revendications 1 à 6, **caractérisé en ce que** les proportions mesurées rentrent dans le ou les composants virtuels avec un exposant réel respectivement attribué.

8. Procédé selon l'une des revendications 1 à 7, **caractérisé en ce que** les coefficients de l'application non linéaire sont déterminés par régression à plusieurs variables en partant d'un modèle prédictif du processus de transformation chimique pour l'approximation du modèle prédictif à l'intérieur d'une fenêtre d'exploitation prévue du processus de transformation.

9. Procédé selon l'une des revendications 1 à 8, **caractérisé en ce que** les coefficients sont dérivés à partir d'un champ caractéristique des compositions de produit escomptées de toutes les configurations de grandeurs d'entrée possibles ou du moins prévues à l'intérieur de la fenêtre d'exploitation du processus de transformation, où le champ caractéristique est généré à partir d'un modèle prédictif du processus de transformation chimique.

10. Procédé selon l'une des revendications 1 à 9, **caractérisé en ce que**, lors de la dérivation des coefficients, toutes les proportions des composants de produit mesurées et/ou mesurables dans le cadre du processus de transformation sont prises en compte.

11. Procédé selon l'une des revendications 1 à 10, **caractérisé en ce que**, lors de la dérivation des coefficients avec les proportions mesurées des composants de produit (C2H4, C2H6, C3H6, C3H8, BUTAD, NBUTA), les erreurs de mesure associées sont prises en compte en donnant de préférence un poids plus important aux proportions mesurables précisément qu'aux proportions mesurables moins précisément.

12. Procédé selon l'une des revendications 1 à 11, **caractérisé en ce que** le processus de transformation chimique est un processus de craquage vapeur, où le produit est un gaz de craquage et les composants de produit sont des composants du gaz de craquage, en particulier les hydrocarbures contenus dans le gaz de craquage.

13. Procédé selon la revendication 12, **caractérisé en ce que** la ou les grandeurs d'entrée (COT ; COP ; FLOWR ; DS) sont et/ou comprennent une température de gaz de craquage (COT) à une sortie du tube de craquage et/ou à une sortie de four, une pression de gaz de craquage (COP) à la sortie du tube de craquage et/ou à la sortie du four, une quantité de mise en oeuvre (FLOWR) d'hydrocarbures à craquer à une entrée du tube de craquage, une composition de mise en oeuvre et/ou un rapport (DS) d'un volume de vapeur de processus sur la quantité de mise en oeuvre.

14. Dispositif destiné à la correction d'une mesure d'une grandeur d'entrée (COT ; COP ; FLOWR ; DS) d'un processus de transformation chimique pour la fabrication d'un produit composé de plusieurs composants de produit (C2H4, C2H6, C3H6, C3H8, BUTAD, NBUTA) d'après le procédé selon l'une des revendications 1 à 13, comprenant des moyens de mesure (12) pour la mesure de la grandeur d'entrée (COT ; COP ; FLOWR ; DS) et pour la mesure des proportions de plusieurs composants de produit (C2H4, C2H6, C3H6, C3H8, BUTAD, NBUTA) dans le produit, lesdits moyens de mesure (12) étant raccordés à des moyens de calcul (13), où les moyens de calcul (13) sont conçus pour déterminer une valeur de correction de la grandeur d'entrée (COT ; COP ; FLOWR ; DS) à l'aide des proportions mesurées, **caractérisé en ce que** les moyens de mesure (12) sont conçus pour mesurer les proportions respectives d'au moins trois composants de produit (C2H4, C2H6, C3H6, C3H8, BUTAD, NBUTA) et **en ce que** les moyens de calcul (13) sont conçus pour calculer la valeur de correction à partir d'une valeur de mesure de la grandeur d'entrée (COT ; COP ; FLOWR ; DS) et d'une valeur actuelle d'un composant virtuel, qui correspond à une application non linéaire d'au moins trois proportions mesurées, où l'application non linéaire est une combinaison linéaire de fonctions de base non linéaires des proportions mesurées, où chaque fonction de base se voit attribuer un coefficient de pondération de la fonction de base par régression linéaire à plusieurs variables en partant d'un modèle prédictif du processus de transformation chimique de telle sorte que, au moins à l'intérieur d'une fenêtre d'exploitation de plusieurs grandeurs d'entrée (COT, COP, FLOWR, DS), l'influence des grandeurs d'entrée restantes sur le composant virtuel soit inférieure à celle de la grandeur d'entrée mesurée (COT ; COP ; FLOWR ; DS).

Fig. 1

Fig. 2

Fig 3

Fig. 3

Fig. 4

Fig 5

## C2H4 [m%]

a)

## C2H6 [m%]

b)

## C3H6 [m%]

c)

Fig 6

C3H8 [m%]

d)

BUTAD [m%]

e)

NBUTA [m%]

f)

Fig 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4127536 A1

- CN 101286059 A

**In der Beschreibung aufgeführte Nicht-Patentliteratur**

- State and parameter estimation in chemical and biochemical processes: a tutorial. *Journal of Process Control,* 01. Dezember 2003, vol. 12 (8), 801-818